(19) Europäisches Patentamt / European Patent Office / Office européen des brevets

(11) **EP 4 129 164 B1**

(12) **EUROPEAN PATENT SPECIFICATION**

(45) Date of publication and mention
of the grant of the patent:
**26.11.2025 Bulletin 2025/48**

(21) Application number: **21788350.3**

(22) Date of filing: **23.03.2021**

(51) International Patent Classification (IPC):
**A61B 5/0205** (2006.01)    **A61B 5/00** (2006.01)

(52) Cooperative Patent Classification (CPC):
**A61B 5/0062; A61B 5/0205; A61B 5/02141;**
**A61B 5/0225; A61B 5/02438; A61B 5/0537;**
**A61B 5/4872; A61B 5/681; A61B 5/742;**
**A61B 5/746;** A61B 5/6802; A61B 5/6803;
A61B 5/6804; A61B 5/6806; A61B 5/6807;   (Cont.)

(86) International application number:
**PCT/CN2021/082381**

(87) International publication number:
**WO 2021/208679 (21.10.2021 Gazette 2021/42)**

(54) **BLOOD PRESSURE DETECTION METHOD AND DEVICE**

BLUTDRUCKDETEKTIONSVERFAHREN UND -VORRICHTUNG

PROCÉDÉ ET DISPOSITIF DE DÉTECTION DE TENSION ARTÉRIELLE

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **16.04.2020 CN 202010301533**

(43) Date of publication of application:
**08.02.2023 Bulletin 2023/06**

(73) Proprietor: **Huawei Technologies Co., Ltd.**
**Shenzhen, Guangdong 518129 (CN)**

(72) Inventors:
• **ZHENG, Chenggong**
**Shenzhen, Guangdong 518129 (CN)**
• **WANG, Shaojian**
**Shenzhen, Guangdong 518129 (CN)**
• **HUANG, Zhenlong**
**Shenzhen, Guangdong 518129 (CN)**

(74) Representative: **Epping - Hermann - Fischer**
**Patentanwaltsgesellschaft mbH**
**Schloßschmidstraße 5**
**80639 München (DE)**

(56) References cited:
| | |
|---|---|
| CN-A- 1 627 916 | CN-A- 109 984 736 |
| CN-A- 110 198 661 | CN-U- 205 285 120 |
| CN-U- 207 152 582 | CN-U- 212 574 862 |
| JP-A- 2002 159 457 | US-A- 5 042 496 |
| US-A1- 2003 149 369 | US-A1- 2010 036 265 |
| US-A1- 2014 163 399 | US-A1- 2016 038 055 |
| US-A1- 2019 290 143 | US-A1- 2019 365 259 |

(52) Cooperative Patent Classification (CPC): (Cont.)
A61B 8/0858; A61B 8/4227; A61B 8/4416

**Description**

This application claims priority to Chinese Patent Application No. 202010301533.9, filed with the China National Intellectual Property Administration on April 16, 2020 and entitled "BLOOD PRESSURE DETECTION METHOD AND APPARATUS'.

**TECHNICAL FIELD**

This application pertains to the field of medical instruments, and in particular, to a blood pressure detection method and apparatus.

**BACKGROUND**

Currently, a mainstream home electronic sphygmomanometer on the market is a wrist electronic sphygmomanometer. For ease of carrying, the wrist electronic sphygmomanometer further evolves into a blood pressure wrist strap, a wrist sphygmomanometer, or a blood pressure watch, to implement blood pressure tracking and monitoring.

When measuring blood pressure at a wrist, the wrist electronic sphygmomanometer measures an air pressure signal in an airbag by using an air pressure sensor connected to the airbag. Ideally, air pressure in the airbag may be delivered to a radial artery without loss through the airbag and a wrist tissue. Therefore, a pulse wave signal can be accurately obtained based on the air pressure signal. However, an actual case is affected by a plurality of factors. For example, in a case of the same wrist circumference, if a subject has relatively thick wrist fat and a radial artery is deeply hidden, because an elastic adipose tissue provides a buffer function, the air pressure in the airbag is greater than pressure actually imposed on the radial artery, and a pulse wave signal moves rightward. Consequently, measured blood pressure is higher than actual systolic blood pressure.

Therefore, for the blood pressure wrist strap, the wrist sphygmomanometer, or the blood pressure watch, when a width and a length of the airbag are fixed, a wrist circumference size and a wrist fat thickness greatly affect the pulse wave signal, causing low accuracy of blood pressure measurement.

US 2019/365259 Al relates to a blood pressure monitor, a blood pressure measurement apparatus, and a blood pressure measurement method. The publication discloses a blood pressure monitor including a pressing member that includes a pressure cuff having a bag shape capable of containing fluid and generates a pressing force toward a measurement site of a subject when the pressing member is wound around the measurement site and the pressure cuff is inflated. The blood pressure monitor further includes a controller that controls an operation of the adjuster such that the fluid is contained in the sensing cuff in an amount corresponding to biometric information about the subject and the pressure cuff is inflated or inflated and deflated in this state when performing a blood pressure measurement, and calculates a blood pressure value of the subject based on an output from the pressure sensor.

US 2014/163399 Al discloses a method and an apparatus for non-invasively assessing one or more hemodynamic parameters associated with the circulatory system of a living organism. The publication describes a method of measuring a hemodynamic parameter by applanating or compressing portions of tissue proximate to the blood vessel of concern until a desired condition is achieved, and then measuring the hemodynamic parameter.

US 2019/290143 Al relates to a sphygmomanometer, and more particularly, a wrist-type sphygmomanometer that includes a main body that is mounted with a pump wherein the main body is to be disposed on a dorsal surface (surface corresponding to a back side of a hand) of an outer circumferential surface of a wrist as a measurement target site and a cuff that extends from the main body and to be mounted around the wrist. The publication also relates to a blood pressure measurement method for measuring a blood pressure at a measurement target site. A fluid is supplied from the pump to the cuff to press the wrist. A blood pressure is calculated based on a pressure of the fluid contained in the cuff.

US 2010/036265 Al discloses a method of measuring blood pressure that includes measuring a characteristic of body fat of a measured body part, detecting a sphygmus wave and a pressure of a blood vessel of the measured body part, detecting a base blood pressure of the measured body part based on a result of the detecting the sphygmus wave and the pressure of the blood vessel of the measured body part, and detecting a continuous blood pressure using a blood pressure calibration. The blood pressure calibration uses the base blood pressure and a blood pressure calculation regression equation including the characteristic of the body fat.

JP 2002 159457 A discloses A body-worn measuring device comprising a sensor that has a piezoelectric element for transmitting which sends ultrasound to the radial artery and a piezoelectric element for receiving which catches reflective waves produced when the radial artery reflects the ultrasound. A belt secures the sensor to a subject. Non-slip parts are fixed to and placed lengthwise along the both sides of the belt where the sensor comes in a manner that the non-slip parts make contact with the surface of the subject's body, and work against shear displacements between the sensor and the surface of the body.

CN 207 152 582 U relates to the technical field of medical equipment, in particular to a human body fat measurement and analysis system. The body fat measurement and analysis system includes a wristband measuring device for collecting and analyzing raw data of a fat thickness of a designated portion of a human body. The ring measuring device includes a measuring body and a connecting band for binding with the wrist, the connecting band is connected to the measuring body, and the measuring body is provided with a near-infrared emitting probe for emitting near-infrared light. a photoelectric sensor for receiving near-infrared light scattered by a human body and converted into an output voltage, a display device for displaying the analysis result, and a key for controlling activation of the wrist-wound measuring device.

US 2016/038055 Al describes a wearable device including a housing and a mount configured to mount the housing to an external surface of a wearer. The wearable devices further include first and second electrical contacts protruding from the housing and configured such that the electrical contacts can be used to measure a Galvanic skin resistance of skin proximate to the electrical contacts when the wearable device is mounted to the external surface of the wearer. The electrical contacts are additionally configured to measure a capacitance between electrical contacts. The measured capacitance between the electrical contacts could be related to a capacitance of skin proximate to the electrical contacts when the wearable device is mounted to the external surface of the wearer.

Document US2003/149369 A1 discloses a method, comprising obtaining, by a wearable device, wrist circumference data, and blood pressure data of a user corresponding to the wearable device; and correcting, by the wearable device, the blood pressure data based on the wrist circumference data.

## SUMMARY

The invention is defined by the appended claims. Embodiments of this application provide a blood pressure detection method and apparatus, to improve accuracy of blood pressure measurement.

According to a first aspect, an embodiment of this application provides a blood pressure detection method, including: A wearable device first obtains wrist circumference data, wrist fat thickness data, and blood pressure data. Then, the wearable device corrects the blood pressure data based on the wrist circumference data and the wrist fat thickness data, wherein the obtaining wrist fat thickness data comprises: inputting test currents on a plurality of frequencies to the wrist of the user; detecting a plurality of electric potential differences formed at the wrist by the test currents on a plurality of frequencies; and determining the wrist fat thickness data based on the plurality of test currents and the plurality of electric potential differences.

The wearable device corrects the blood pressure data based on the wrist circumference data and the wrist fat thickness data, to avoid an impact of a wrist circumference size and a wrist fat thickness on a pulse wave signal, thereby improving accuracy of blood pressure measurement. In addition, the same airbag can be adapted to different people with different wrist circumferences and different wrist fat thicknesses. Target blood pressure can be accurately detected for different users without replacing different types of airbags, to improve compatibility of a blood pressure measurement apparatus and enhance user wearing experience.

In a possible implementation of the first aspect, the blood pressure data may be corrected based on a mapping relationship. The mapping relationship may be a linear fitting function.

For example, the blood pressure data is corrected by using the linear fitting function, and then the corrected blood pressure data is displayed.

It should be understood that the linear fitting function is merely an optional implementation. A possible implementation of the first aspect includes a nonlinear fitting function, for example, a polynomial fitting function. With reference to the first aspect, in an implementation of the first aspect, obtaining the wrist circumference data includes:

first, detecting an electrical parameter of an adjustable component around the wrist of the user, where the adjustable component includes at least one of an adjustable resistor, an adjustable capacitor, or an adjustable inductor; and then obtaining the wrist circumference data corresponding to the electrical parameter.

Because the wrist circumference data is detected based on the electrical parameter of the adjustable component around the wrist of the user, portability for detection of the wrist circumference data is improved, and the costs of the wrist circumference data are reduced.

With reference to the first aspect, in an implementation of the first aspect, after the wearable device corrects the blood pressure data based on the wrist circumference data and the wrist fat thickness data, the method further includes: The wearable device first determines a health level of the user based on the wrist fat thickness data and the detected blood pressure data; and then the wearable device displays the health level.

Because the health level is obtained based on the wrist fat thickness data and the detected blood pressure data, an indicator of the health of the user is comprehensively considered, and accuracy of the health level is improved.

With reference to the first aspect, in an implementation of the first aspect, after the health level of the user is determined, the method further includes:

The wearable device obtains a total calorie intake of the user within unit time; the wearable device obtains total calorie consumption of the user within the unit time; and finally, the wearable device outputs health prompt information based on the total calorie intake, the total calorie consumption, and the health level.

Because the health level is considered when the health prompt information is pushed, the health prompt information is more specific, and the user can obtain the health prompt information matching the personal health level.

With reference to the first aspect, in an implementation of the first aspect, that the wearable device corrects the blood pressure data based on the wrist circumference data and the wrist fat thickness data includes:

First, when the wearable device determines, based on the wrist circumference data and the wrist fat thickness data, that the blood pressure data needs to be corrected, the wearable device obtains, from a preset database, a mapping relationship corresponding to the wrist circumference data and the wrist fat thickness data. The mapping relationship is the mapping relationship corresponding to the wrist circumference data and the wrist fat thickness data. The wearable device corrects the blood pressure data based on the mapping relationship, and displays corrected blood pressure data.

The wearable device obtains, from the preset database, the mapping relationship corresponding to the wrist circumference data and the wrist fat thickness data; and then corrects the blood pressure data based on the mapping relationship, and uses the corrected blood pressure data as the detected blood pressure data. Because the mapping relationship corresponds to the wrist circumference data and the wrist fat thickness data, the blood pressure data is corrected based on the mapping relationship, to further improve accuracy of blood pressure measurement, improve compatibility of a blood pressure measurement apparatus, and enhance user wearing experience.

According to a second aspect, an embodiment of this application provides a blood pressure detection apparatus, including:

a main body part and a strap connected to the main body part, where the strap is configured to wear the main body part on a wrist of the user, and the processor is disposed in the main body part; and a wrist circumference detection component, a wrist fat thickness detection component, a blood pressure detection component, and a processor, where the processor is disposed in the main body part and is separately connected to the wrist circumference detection component, the wrist fat thickness detection component, and the blood pressure detection component.

The wrist circumference detection component is configured to obtain wrist circumference data of a user.

The wrist fat thickness detection component is configured to obtain wrist fat thickness data of the user. The wrist fat thickness detection component comprises: an excitation electrode, a detection electrode, and an impedance detection circuit, wherein the excitation electrode is disposed on a side surface of the main body part and is configured to forward an excitation voltage; the detection electrode is disposed at a bottom of the main body part and is configured to receive a detection voltage, wherein the detection voltage is generated based on the excitation voltage and a voltage drop of wrist fat of the user; and the impedance detection circuit is disposed in the main body part and is configured to: generate the excitation voltage, and obtain the wrist fat thickness data through calculation based on the excitation voltage and the detection voltage

The blood pressure detection component is configured to obtain blood pressure data of the user.

The processor is configured to correct the blood pressure data based on the wrist circumference data and the wrist fat thickness data.

The processor corrects the blood pressure data based on the wrist circumference data and the wrist fat thickness data, to avoid an impact of a wrist circumference size and a wrist fat thickness on blood pressure measurement, thereby improving accuracy of blood pressure measurement. In addition, a same airbag can be adapted to different people with different wrist circumferences and different wrist fat thicknesses. Target blood pressure can be accurately detected for different users without replacing different types of airbags, to improve compatibility of the blood pressure measurement apparatus and enhance user wearing experience.

With reference to the second aspect, in an implementation of the second aspect, the wrist circumference detection component includes:

an adjustable component and an electrical parameter detection circuit, where the adjustable component is connected to the electrical parameter detection circuit, and the electrical parameter detection circuit is disposed in the main body part and is configured to: detect an electrical parameter of the adjustable component, and obtain, based on a preset correspondence between the electrical parameter and the wrist circumference data, wrist circumference data corresponding to the detected electrical parameter.

The wrist circumference data is detected by using the adjustable component and the electrical parameter detection circuit. In this way, portability for detection of the wrist circumference data is improved, and the costs of the wrist circumference data are reduced.

The wrist fat thickness data is detected by using the excitation electrode, the detection electrode, and the impedance detection circuit. A non-traumatic wrist fat thickness data detection manner is provided, and accuracy of the wrist fat thickness data is improved.

With reference to the second aspect, in an implementation of the second aspect, the blood pressure detection component includes:

an air pump, an airbag, and a pressure sensor, where the air pump and the pressure sensor are disposed in the main body part, the airbag is disposed on a second surface of the strap, and the second surface is a contact surface of the strap with the wrist when the blood pressure detection apparatus is worn on the wrist of the user, the air pump is connected to the airbag, and the airbag is connected to the pressure sensor.

The air pump is configured to inflate the airbag. The pressure sensor is configured to: in a process of inflating the airbag, detect air pressure in the airbag in real time, and obtain the blood pressure data through calculation based on the air pressure.

With reference to the second aspect, in an implementation of the second aspect, the blood pressure detection apparatus further includes:

an indication component, where the indication component is disposed on a third surface of the main body part, the indication component is connected to the processor, the third surface is a surface away from the wrist when the blood pressure detection apparatus is worn on the wrist of the user, and the indication component is configured to prompt a health condition of the user by using an indication signal.

The health condition of the user is prompted by using the indication signal, to intuitively indicate the health condition of the user and improve user experience of the blood pressure detection apparatus.

It may be understood that, for the beneficial effects of the second aspect, refer to the related description in the first aspect. Details are not described herein again.

In embodiments of this application, the wearable device corrects the blood pressure data based on the wrist circumference data and the wrist fat thickness data, to avoid an impact of a wrist circumference size and a wrist fat thickness on blood pressure measurement, thereby improving accuracy of blood pressure measurement. In addition, the same airbag can be adapted to different people with different wrist circumferences and different wrist fat thicknesses. Blood pressure data can be accurately detected for different users without replacing different types of airbags, to improve compatibility of the blood pressure measurement apparatus and enhance user wearing experience.

**BRIEF DESCRIPTION OF DRAWINGS**

FIG. 1 is a block diagram of a module principle of a blood pressure detection apparatus according to an embodiment of this application;
FIG. 2 is a block diagram of a module principle of a wrist circumference detection circuit in a blood pressure detection apparatus according to an embodiment of this application;
FIG. 3 is a schematic diagram of a structure of an embedded adjustable component of a first strap in a buckle design of a blood pressure detection apparatus according to an embodiment of this application;
FIG. 4 is a side view of a second strap in a buckle design of a blood pressure detection apparatus according to an embodiment of this application;
FIG. 5 is a front view of a second strap in a buckle design of a blood pressure detection apparatus according to an embodiment of this application;
FIG. 6 is a schematic diagram of a structure of holes in a strap in a buckle design of a blood pressure detection apparatus according to an embodiment of this application;

FIG. 7 is a side view of a strap in a butterfly clasp design of a blood pressure detection apparatus according to an embodiment of this application;
FIG. 8 is an enlarged schematic diagram of a butterfly clasp 213 in FIG. 18;
FIG. 9 is a front view of a strap in a butterfly clasp design of a blood pressure detection apparatus according to an embodiment of this application;
FIG. 10 is a block diagram of another module principle of a wrist fat thickness detection circuit in a blood pressure detection apparatus according to an embodiment of this application;
FIG. 11 is a schematic diagram of a structure of side electrodes of a main body part of a blood pressure detection apparatus according to an embodiment of this application;
FIG. 12 is a schematic diagram of a structure of bottom electrodes of a main body part of a blood pressure detection apparatus according to an embodiment of this application;
FIG. 13 is a block diagram of another module principle of a blood pressure detection apparatus according to an embodiment of this application;
FIG. 14 is a schematic flowchart of a blood pressure detection method according to an embodiment of this application;
FIG. 15 is a relationship diagram indicating that a frequency of a test current changes with time in a process of performing impedance detection on a wrist of a user to obtain wrist fat thickness data;
FIG. 16 is a schematic flowchart of another blood pressure detection method according to an embodiment of this application;
FIG. 17 is a schematic flowchart of another blood pressure detection method according to an embodiment of this application;
FIG. 18 is a schematic diagram of an interface for displaying a health level;
FIG. 19 is a schematic diagram of an interface of a blood pressure watch;
FIG. 20 is a schematic diagram of a structure of a blood pressure detection apparatus according to an embodiment of this application;
FIG. 21 is a schematic diagram of another structure of a blood pressure detection apparatus according to an embodiment of this application;
FIG. 22 is a schematic diagram of another structure of a blood pressure detection apparatus according to an embodiment of this application;
FIG. 23 is a schematic diagram of another structure of a correction module in a blood pressure detection apparatus according to an embodiment of this application;
FIG. 24 is a schematic diagram of another structure of a correction module in a blood pressure detection apparatus according to an embodiment of this application; and
FIG. 25 is a schematic diagram of another structure of a blood pressure detection apparatus according to an embodiment of this application.

## DESCRIPTION OF EMBODIMENTS

In the following description, to illustrate rather than limit, specific details such as a particular system structure and a technology are provided to make a thorough understanding of embodiments of this application. However, persons skilled in the art should know that this application may be practiced in other embodiments without these specific details. In other cases, detailed descriptions of well-known systems, apparatuses, circuits, and methods are omitted, so that this application is described without being obscured by unnecessary details.

It should be understood that the term "include" used in this specification and the appended claims in this application specifies presence of features, integers, steps, operations, elements, and/or components, with presence or addition of one or more other features, integers, steps, operations, elements, components, and/or their combinations not excluded. It should be further understood that the term "and/or" used in this specification and the appended claims in this application means any combination of one or more of the associated listed items and all possible combinations and includes these combinations.

According to the context, the term "if" used in this specification and the appended claims in this application may be interpreted as a meaning of "when" or "once" or "in response to determining" or "in response to detecting". Similarly, according to the context, the phrase "if it is determined that" or "if (a stated condition or event) is detected" may be interpreted as a meaning of "once it is determined that" or "in response to determining" or "once (a stated condition or event) is detected" or "in response to detecting (a stated condition or event)".

In addition, in the description of this specification and the appended claims in this application, the terms "first", "second", "third", and the like are merely used to distinguish the description, and cannot be construed as indicating or implying relative importance.

Reference to "an embodiment", "some embodiments", or the like described in this specification in this application indicates that one or more embodiments of this application include a specific feature, structure, or characteristic described with reference to embodiments. Therefore, statements such as "in an embodiment", "in some embodiments", "in some other embodiments", and "in other embodiments" that appear at different places in this specification do not necessarily mean referring to a same embodiment. Instead, the statements mean "one or more but not all of embodiments", unless otherwise specifically emphasized in another manner. The terms "include", "contain", "have", and their variants all mean "include but are not limited to", unless otherwise specifically emphasized in another manner. The blood pressure detection method provided in this embodiment of this application may be applied to an electronic device, for example, a wearable device. A specific type of the wearable device is not limited in embodiments of this application.

As an example instead of limitation, when the electronic device is a wearable device, the wearable device may be a generic term of wearable devices, for example, glasses, gloves, watches, clothes, and shoes, developed through implementing an intelligent design on daily wear by using a wearable technology. The wearable device is a portable device that is directly worn on a body or integrated into clothes or an accessory of a user. The wearable device is not merely a hardware device, but is used to implement a powerful function through software support, data interaction, and cloud interaction. In a board sense, wearable intelligent devices include full-featured and large-sized devices that can implement complete or partial functions without depending on smartphones, for example, smart watches or smart glasses, and devices that focus on only one type of application function and need to work with other devices such as smartphones, such as various smart bands, smart watches, or smart jewelry for monitoring physical signs.

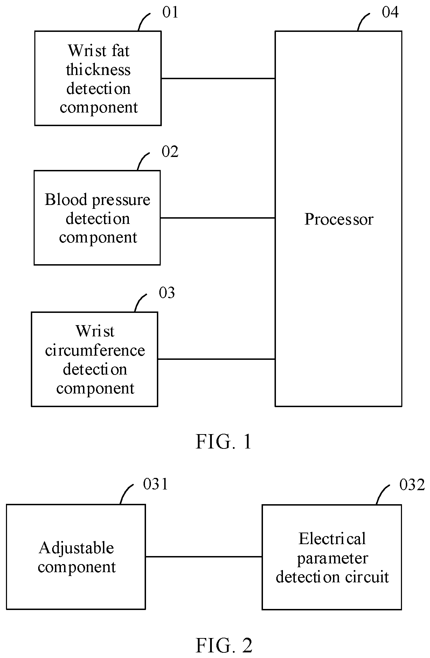

FIG. 1 shows a structure of a blood pressure detection apparatus provided in an embodiment of this application. For ease of description, only a part related to this embodiment of the present invention is shown. Details are as follows:

The blood pressure detection apparatus includes: a wrist circumference detection component 03, a wrist fat thickness detection component 01, a blood pressure detection component 02, and a processor 04. The processor 04 is separately connected to the wrist circumference detection component 03, the wrist fat thickness detection component 01, and the blood pressure detection component 02.

The wrist circumference detection component 03 is configured to obtain wrist circumference data of a user. The wrist fat thickness detection component 01 is configured to obtain wrist fat thickness data of the user. The blood pressure detection component 02 is configured to obtain blood pressure data of the user. The processor 04 is configured to correct the blood pressure data based on the wrist circumference data and the wrist fat thickness data.

The processor 04 is specifically configured to: determine, based on the wrist circumference data and the wrist fat thickness data, whether the blood pressure data needs to be corrected, and when the blood pressure data needs to be corrected, obtain, from a preset database, a mapping relationship corresponding to the wrist circumference data and the wrist fat thickness data, and correct the blood pressure data based on the mapping relationship.

The blood pressure detection apparatus includes: a main body part and a strap connected to the main body part. The strap is configured to wear the main body part on a wrist of the user. The processor 04 is disposed in the main body part.

As shown in FIG. 2, the wrist circumference detection component 03 includes an adjustable component 031 and an electrical parameter detection circuit 032. The adjustable component 031 is connected to the electrical parameter detection circuit 032. The electrical parameter detection circuit 032 is disposed in the main body part and is configured to: detect an electrical parameter of the adjustable component, and obtain, based on a preset correspondence between the electrical parameter and the wrist circumference data, wrist circumference data corresponding to the detected electrical parameter.

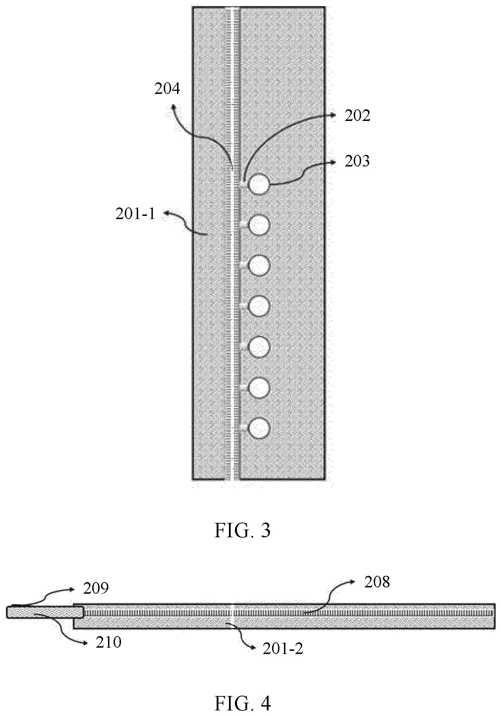

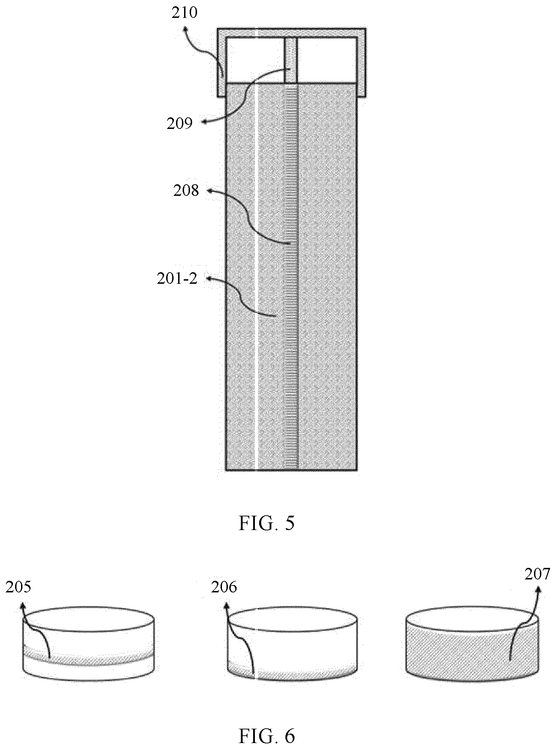

In an implementation, the strap may use a buckle design. For example, in FIG. 3 to FIG. 6, the strap includes a first strap 201-1 and a second strap 201-2. The first strap 201-1 and the second strap 201-2 are separately connected to the main body part. The adjustable component 204 is disposed on a first surface of the first strap 201-1. The first surface is a surface close to the wrist when the blood pressure detection apparatus is worn on the wrist of the user. The first strap 201-1 is further provided with a plurality of holes 203 parallel to the adjustable component 204. The adjustable component 204 is connected to the holes 203 through a first conducting wire 202. The second strap 201-2 is provided with a buckle tongue 209 matching the holes 203. A second conducting wire 205 is disposed on both surfaces of the buckle tongue 209 and the holes 203. When the buckle tongue 209 is connected to the holes 203 through the second conducting wire 205, an electrical parameter of the adjustable component 204 may be adjusted based on a position of the buckle tongue 209.

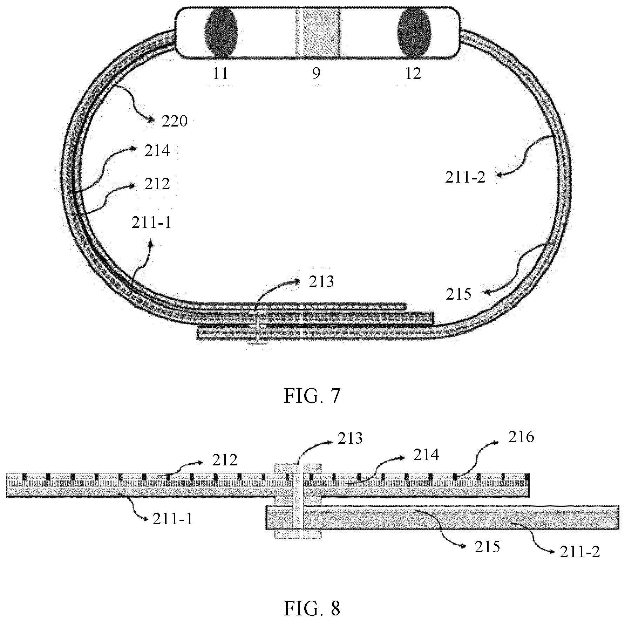

In another implementation, the strap may alternatively use a butterfly clasp design. For example, in FIG. 7 to FIG. 9, the strap includes a first strap 211-1 and a second strap 211-2. The first strap 211-1 and the second strap 211-2 are separately connected to the main body part. The adjustable component 214 is disposed on a first surface of the first strap 211-2. The first surface is a surface close to the wrist when the blood pressure detection apparatus is worn on the wrist of the user. An insulator 212 is disposed on a surface of the adjustable component 214. A plurality of third conducting wires 216 are embedded in the insulator 212 in a spaced manner and connected to the adjustable component 214. A movable butterfly clasp 213 is disposed on the second strap 211-2. The butterfly clasp 213 is connected to the adjustable component 214 through the third conducting wires 216. When the butterfly clasp 213 moves on the strap, an electrical parameter of the adjustable component 214 may be adjusted based on a position of the butterfly clasp 213.

Optionally, the adjustable component includes at least one of an adjustable resistor, an adjustable capacitor, and an adjustable inductor.

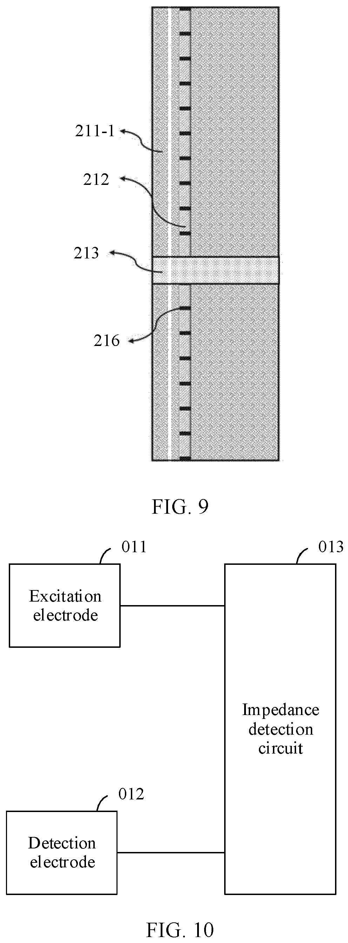

As shown in FIG. 10, the wrist fat thickness detection component 01 includes an excitation electrode 011, a detection electrode 012, and an impedance detection circuit 013.

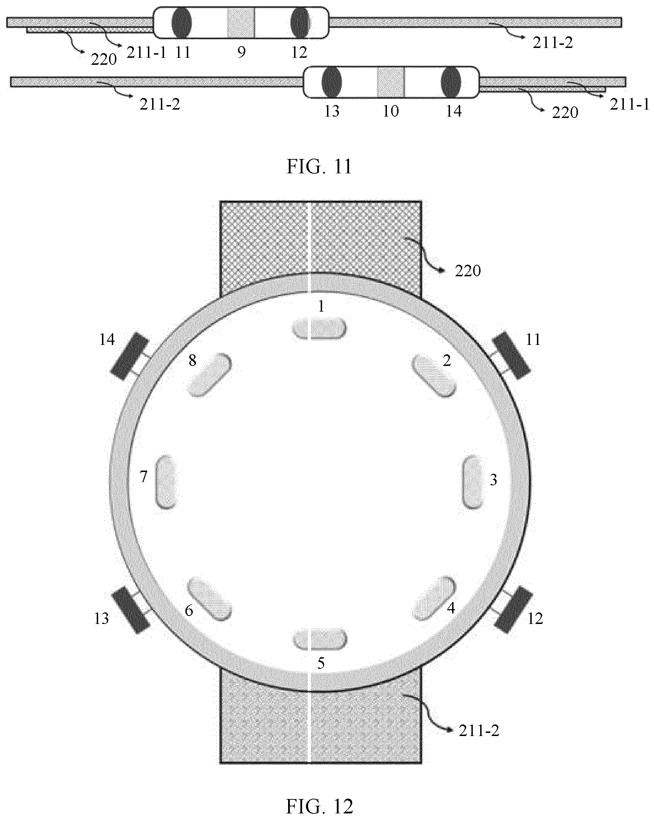

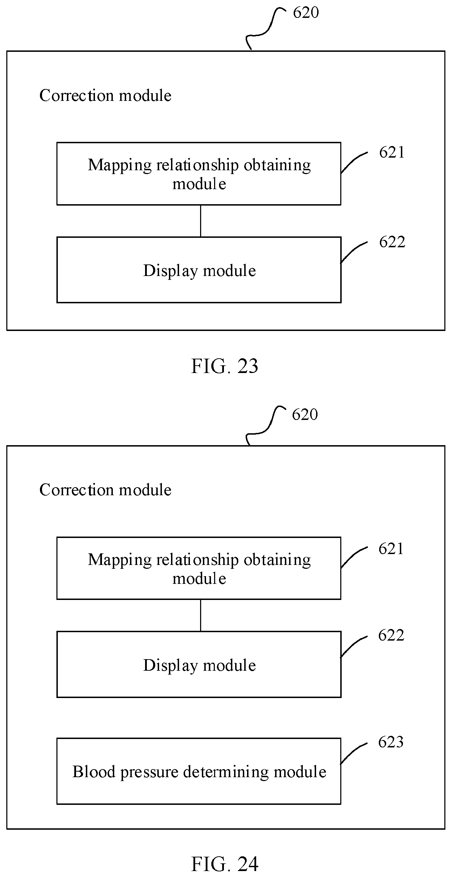

As shown in FIG. 11, the excitation electrode 011 is disposed on a side surface of the main body part and is configured to forward an excitation voltage. As shown in FIG. 23, the detection electrode 012 is disposed at a bottom of the main body part and is configured to receive a detection voltage. The detection voltage is generated based on the excitation voltage and a voltage drop of wrist fat of the user. The impedance detection circuit 013 is disposed in the main body part and is configured to: generate the excitation voltage, and obtain the wrist fat thickness data through calculation based on the excitation voltage and the detection voltage.

A non-adipose tissue has relatively low electrical impedance because the non-adipose tissue contains many electrolytes and water. An adipose tissue is anhydrous and has relatively high electrical impedance. The impedance detection circuit 013 obtains bioelectrical impedance based on the excitation voltage and the detection voltage. The voltage drop of the wrist fat of the user indicates an electric potential difference generated due to the bioelectrical impedance. The impedance detection circuit 013 may further obtain the wrist circumference fat thickness data based on the bioelectrical impedance.

Optionally, as shown in FIG. 11 and FIG. 12, there are a plurality of excitation electrodes 011. The excitation electrodes 011 are sequentially arranged on the side surface of the main body part. There are a plurality of detection electrodes 012. The detection electrodes 012 are disposed at the bottom of the main body part and are sequentially arranged along the periphery of the bottom of the main body part. Each excitation electrode is connected to one detection electrode. The plurality of excitation electrodes 011 and the plurality of detection electrodes 012 are disposed, so that the impedance detection circuit 013 can generate a wrist fat thickness detection signal based on a plurality of excitation voltages and a plurality of detection voltages, to more accurately determine the wrist fat thickness data, thereby improving accuracy of blood pressure detection.

In FIG. 11 and FIG. 12, a button 11, a button 12, a button 13, and a button 14 are separately disposed on the side surface of the main body part. Eight detection electrodes disposed at the bottom of the main body part are respectively a detection electrode 1, a detection electrode 2, a detection electrode 3, a detection electrode 4, a detection electrode 5, a detection electrode 6, a detection electrode 7, and a detection electrode 8. Two excitation electrodes are respectively disposed on left and right side of the main body part, respectively: an excitation electrode 9 and an excitation electrode 10. The detection electrode 1, the detection electrode 3, the detection electrode 5, and the detection electrode 7 are connected to form a first group of detection electrodes; and the detection electrode 2, the detection electrode 4, the detection electrode 6, and the detection electrode 8 are connected to form a second group of detection electrodes. The first group of detection electrodes are connected to the excitation electrode 9 by using the human body of the user, and the second group of detection electrodes are connected to the excitation electrode 10 by using the human body of the user.

It is assumed that the blood pressure detection apparatus is worn on the left hand, the electrode at the bottom of the main body part of the blood pressure detection apparatus is in contact with the left wrist, and two fingers of the right hand are in contact with two electrodes on the side surface of the main body part. In this case, the human body accesses a circuit. Due to conductivity of the human body, the first group of detection electrodes are connected to the excitation electrode 9 by using the human body of the user, and the second group of detection electrodes are connected to the excitation electrode 10 by using the human body of the user to form a loop. In addition, impedance of the human body is not 0. Therefore, there is an electric potential difference between electrodes. The impedance detection circuit 013 may calculate the wrist fat thickness data based on an electric potential difference between each of the plurality of excitation voltages and each of the plurality of detection voltages.

It should be noted that a position of the excitation electrode 011 and a position of the detection electrode 012 may be interchanged. The plurality of excitation electrodes 011i may also be disposed at the bottom of the main body part of the blood pressure detection apparatus and sequentially arranged along the periphery of the bottom of the main body part of the blood pressure detection apparatus. The plurality of groups of detection electrodes 012i may also be sequentially arranged on the side surface of the main body part of the blood pressure detection apparatus.

In another implementation, the wrist fat thickness detection circuit 01 includes at least one of an optical sensor or an ultrasonic sensor.

The wrist fat thickness detection circuit 01 may transmit a first ultrasonic wave to the wrist of the user, and record transmit time of the first ultrasonic wave. When receiving a second ultrasonic wave, the wrist fat thickness detection circuit 01 records receive time of the second ultrasonic wave. The second ultrasonic wave is an ultrasonic wave reflected after the first ultrasonic wave reaches the bone of the wrist. The wrist fat thickness detection circuit 01 determines the wrist fat thickness data based on the transmit time of the first ultrasonic wave and the receive time of the second ultrasonic wave.

It should be noted that the blood pressure detection component includes an air pump, an airbag, and a pressure sensor. The air pump and the pressure sensor are disposed in the main body part. As shown in FIG. 11 and FIG. 12, the airbag 220 is disposed on a second surface of the strap 211-1. The second surface is a contact surface of the strap with the wrist when the blood pressure detection apparatus is worn on the wrist of the user. The air pump is connected to the airbag 220. The airbag 220 is connected to the pressure sensor. The air pump is configured to inflate the airbag 220. The pressure sensor is configured to: detect air pressure in the airbag in real time in an inflation process of the airbag 220, and obtain the blood pressure data through calculation based on the air pressure.

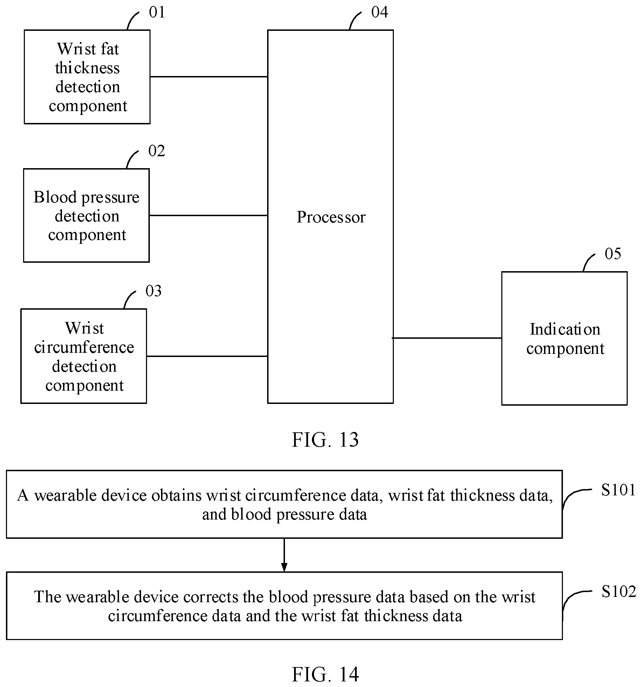

Optionally, as shown in FIG. 13, the blood pressure detection apparatus further includes an indication component 05. The indication component 05 is disposed on a third surface of the main body part. The indication component 05 is connected to the processor 04. The third surface is a surface away from the wrist when the blood pressure detection apparatus is worn on the wrist of the user. The indication component is configured to indicate a health condition of the user by using an indication signal.

The indication component 05 may include a plurality of LEDs. The LEDs blink or glow to indicate whether target blood pressure, a heart rate, and a body fat thickness coefficient of the user are normal. Alternatively, different health conditions may be indicated by using colors (red, orange, yellow, green, and the like) of the LEDs. For example, a red LED indicates a serious problem of the body of the user, an orange LED indicates a relatively large problem of the body of the user, a yellow LED indicates a slight problem of the body of the user, and a green LED indicates a good health condition of the body of the user.

The blood pressure detection apparatus may be a blood pressure wrist strap, a wrist sphygmomanometer, or a blood pressure watch.

A blood pressure detection method provided in an embodiment of this application is described below in detail. With reference to a flowchart of a blood pressure detection method shown in FIG. 14, the method includes the following content.

FIG. 14 is a schematic flowchart of a blood pressure detection method according to this application. As an example instead of limitation, the method may be applied to the foregoing electronic device. The blood pressure detection method includes the following steps: S101: A wearable device obtains wrist circumference data, wrist fat thickness data, and blood pressure data.

Specifically, the wrist circumference data may be a product of a wrist circumference length and a preset coefficient. In a possible implementation, that the wearable device obtains the wrist circumference data of a user may include:

S101-1a: The wearable device detects an electrical parameter of an adjustable component around a wrist of the user. The adjustable component includes at least one of an adjustable resistor, an adjustable capacitor, or an adjustable inductor.

The adjustable component may be disposed in a strap of the electronic device or at a first surface of a strap of the electronic device, and may adjust the electrical parameter of the adjustable component based on a position of a buckle or a butterfly clasp of the strap.

In specific implementation, detection of the electrical parameter includes two cases. In a first case, a constant voltage source is used as a power supply. Different currents flow through the adjustable component in cases of different positions of the buckle or the butterfly clasp of the strap, to obtain different electrical parameters. In a second case, a constant current source is used as a power supply. Different electric potential differences between two ends of the adjustable component are obtained in cases of different positions of the buckle or the butterfly clasp of the strap, to obtain different electrical parameters.

For example, when the electronic device is a wrist electronic device, the electronic device includes a main body part and a strap connected to the main body part. The strap is used to wear the electronic device on the wrist of the user. Optionally, a structural relationship between the adjustable component and the strap is shown in FIG. 3 to FIG. 9. S101-2a: Obtain wrist circumference data corresponding to the electrical parameter.

Specifically, the wrist circumference data corresponding to the electrical parameter may be obtained from a second preset database, or the wrist circumference data corresponding to the electrical parameter may be obtained based on a function relationship.

It should be noted that the adjustable component may include at least one of an adjustable resistor, an adjustable capacitor, and an adjustable inductor. The electrical parameter includes at least one of a capacitance value, a resistance value, and an inductance value.

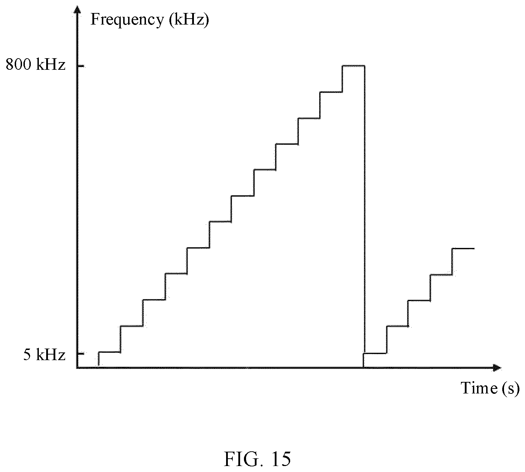

Two possible implementations of obtaining the wrist fat thickness coefficient are specifically as follows: In accordance with the present invention, impedance detection is performed on the human body of the user to obtain a wrist fat thickness coefficient. Specifically, test currents on a plurality of frequencies are input to the user; a plurality of electric potential differences formed in the human body by the test currents on the plurality of frequencies are detected; and the wrist fat thickness data is determined based on the plurality of test currents and the plurality of electric potential differences. FIG. 15 is a relationship diagram indicating that a frequency of a test current changes with time. In FIG. 15, an X-axis represents time, and a Y-axis represents a frequency of a test current.

A non-adipose tissue has relatively low electrical impedance because the non-adipose tissue contains many electrolytes and water. An adipose tissue is anhydrous and has relatively high electrical impedance. In addition, when a direct current or a low-frequency current enters a biological tissue, the current bypasses cells and mainly flows through extracellular fluid. With an increase of a frequency of the current, the current may pass through cell membranes and flow through intracellular fluid. Therefore, the bioelectrical impedance of the wrist of the user changes with the frequency. The plurality of frequencies and an impedance spectrum corresponding to the plurality of frequencies indicate abundant impedance and human body composition information. Therefore, bioelectrical impedance on different frequencies may be obtained based on the plurality of test currents and the plurality of electric potential differences. In this way, the wrist fat thickness data may be determined based on the bioelectrical impedance on different frequencies.

In another possible implementation, ultrasonic distance detection is performed on the wrist of the user to obtain the wrist fat thickness data. A first ultrasonic wave is transmitted to the wrist of the user, and transmit time of the first ultrasonic wave is recorded. When a second ultrasonic wave is received, receive time of the second ultrasonic wave is recorded. The second ultrasonic wave is an ultrasonic wave reflected after the first ultrasonic wave reaches the bone of the wrist. The wrist fat thickness data is determined based on the transmit time of the first ultrasonic wave and the receive time of the second ultrasonic wave.

It should be noted that the wrist circumference data and the wrist fat thickness data may be data detected in real time. For example, when blood pressure detection information is triggered, current wrist circumference data and current wrist fat thickness data of the user are detected.

Preferably, because the wrist circumference data and the wrist fat thickness data of the user change slightly in relatively short time, a time interval may be set, so that the wrist circumference data and the wrist fat thickness data of the user are detected when a specific time interval is reached, and the detected wrist circumference data and the detected wrist fat thickness data are stored. When a next time interval is reached, the wrist circumference data and the wrist fat thickness data that are previously stored are replaced with latest detected wrist circumference data and latest detected wrist fat thickness data. When blood pressure detection information is triggered, the wrist circumference data and the wrist fat thickness data are directly obtained in a storage position.

Finally, the blood pressure data of the user may be obtained by using a sphygmomanometer. Specifically, an air pressure sensor connected to an airbag measures air pressure in the airbag, separates a pulse wave signal from the air pressure, and performs a series of processing on the pulse wave signal, for example, extracts a pulse wave envelope feature and a single pulse wave feature to obtain a feature parameter, and calculates the blood pressure data based on the feature parameter.

S102: The wearable device corrects the blood pressure data based on the wrist circumference data and the wrist fat thickness data.

S102-1: When the wearable device determines, based on the wrist circumference data and the wrist fat thickness data, that the blood pressure data needs to be corrected, the wearable device obtains, from a preset database, a mapping relationship corresponding to the wrist circumference data and the wrist fat thickness data.

Specifically, if the wrist circumference data is not less than preset wrist circumference data and/or the wrist fat thickness data is not less than preset wrist fat thickness data, the wearable device determines that the blood pressure data needs to be corrected.

Step S102-1 may be specifically as follows:

When the wrist fat thickness data is less than a first preset wrist fat thickness, and the wrist circumference data is less than a second wrist circumference, the wearable device determines that the blood pressure data does not need to be corrected.

When the wrist fat thickness data is greater than the first preset wrist fat thickness, and the wrist circumference data is less than a first wrist circumference, the wearable device determines that the blood pressure data does not need to be corrected.

When the wrist fat thickness data is less than the first preset wrist fat thickness, and the wrist circumference data is greater than the second wrist circumference, the wearable device determines that the blood pressure data needs to be corrected.

When the wrist fat thickness data is less than a second preset wrist fat thickness and greater than the first preset wrist fat thickness, and the wrist circumference data is greater than the first wrist circumference and less than the second wrist circumference, the wearable device determines that the blood pressure data needs to be corrected.

When the wrist fat thickness data is less than the second preset wrist fat thickness and greater than the first preset wrist fat thickness, and the wrist circumference data is greater than the second wrist circumference, the wearable device determines that the blood pressure data needs to be corrected.

When the wrist fat thickness data is greater than the second preset wrist fat thickness and greater than the first preset wrist fat thickness, and the wrist circumference data is greater than the first wrist circumference, the wearable device determines that the blood pressure data needs to be corrected.

The first preset wrist fat thickness is less than the second preset wrist fat thickness, and the first wrist circumference is less than the second wrist circumference. The first wrist circumference may be 150 mm, and the second wrist circumference may be 180 mm.

After determining that the blood pressure data needs to be corrected, the wearable device obtains, from the preset database, the mapping relationship matching the wrist circumference data and the wrist fat thickness data. The mapping relationship in the preset database is in one-to-one correspondence with wrist circumference data and wrist fat thickness data.

S102-2: The wearable device corrects the blood pressure data based on the mapping relationship, and displays the corrected blood pressure data.

Specifically, a compensation value is obtained based on the mapping relationship. The blood pressure data is corrected based on the compensation value (a sum of the compensation value and the blood pressure data is used as the corrected blood pressure data). The corrected blood pressure data is displayed.

The mapping relationship may be a quadratic polynomial function or a cubic polynomial function.

For example, the mapping relationship may be

$$y = a_1 x_1^2 + a_2 x_2^2 + a_3 x_1 x_2 + a_4 x_1 + a_5 x_2 + a_6 .$$

Herein, $y$ is the compensation value, $x_1$ is the wrist circumference data, $x_2$ is the wrist fat thickness data, and $a_1$, $a_2$, $a_3$, $a_4$, $a_5$, and $a_6$ are all constants.

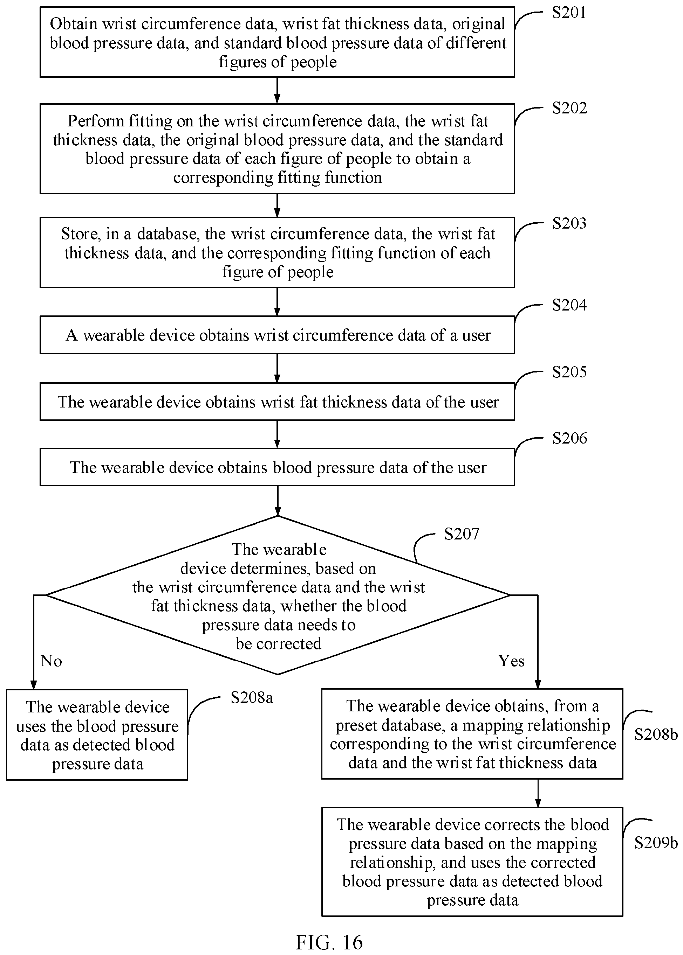

FIG. 16 is a schematic flowchart of another blood pressure detection method according to an embodiment of this application. Details are as follows:

S201: Obtain wrist circumference data, wrist fat thickness data, original blood pressure data, and standard blood pressure data of different figures of people.

First, a plurality of samples are selected for each figure of people. A plurality of pieces of wrist circumference data, wrist fat thickness data, original blood pressure data, and standard blood pressure data are separately measured. Then, data obtained in the experiment is screened to remove an unqualified sample. Finally, an average value is calculated for the plurality of pieces of original blood pressure data of each sample that are measured by a plurality of first sphygmomanometers, and an average value is calculated for the plurality of pieces of standard blood pressure data of each sample that are measured by a plurality of second sphygmomanometers, to form a data point on a calibration curve. The first sphygmomanometer uses a common airbag. The second sphygmomanometer uses an airbag adapted to the wrist circumference data and the wrist fat thickness data of the user; or the second sphygmomanometer is a mercury sphygmomanometer, or a medical arm-type electronic sphygmomanometer. Types of different figures of people include at least one of skin color, gender, age, height, weight, wrist circumference, blood pressure, and various diseases.

S202: Perform fitting on the wrist circumference data, the wrist fat thickness data, the original blood pressure data, and the standard blood pressure data of each figure of people to obtain a corresponding mapping relationship.

A scatter chart is drawn based on the foregoing data points. A plurality of mapping relationships are obtained through function curve fitting based on different wrist circumference data and different wrist fat thickness data. The mapping relationship may be fitted to any function, including a linear function or a non-linear function.

S203: Store, in a database, the wrist circumference data, the wrist fat thickness data, and the corresponding mapping relationship of each figure of people.

The mapping relationship in the database is in one-to-one correspondence with wrist circumference data and wrist fat thickness data.

S204: The wearable device obtains the wrist circumference data of the user.

S205: The wearable device obtains the wrist fat thickness data of the user.

S206: The wearable device obtains the blood pressure data of the user.

S207: The wearable device determines, based on the wrist circumference data and the wrist fat thickness data, whether the blood pressure data needs to be corrected.

Specifically, it is determined whether the wrist circumference data is less than preset wrist circumference data, and whether the wrist fat thickness data is less than preset wrist fat thickness data.

S208a: If the blood pressure data does not need to be corrected, the wearable device uses the blood pressure data as detected blood pressure data.

Specifically, if the wrist circumference data is less than the preset wrist circumference data and the wrist fat thickness data is less than the preset wrist fat thickness data, the wearable device determines that the blood pressure data does not need to be corrected, and uses the blood pressure data as the detected blood pressure data.

S208b: If the blood pressure data needs to be corrected, the wearable device obtains, from a preset database, the mapping relationship corresponding to the wrist circumference data and the wrist fat thickness data.

The mapping relationship matching the wrist circumference data and the wrist fat thickness data is obtained from the preset database. The mapping relationship in the preset database is in one-to-one correspondence with wrist circumference data and wrist fat thickness data.

S209b: The wearable device corrects the blood pressure data based on the mapping relationship, and uses the corrected blood pressure data as detected blood pressure data.

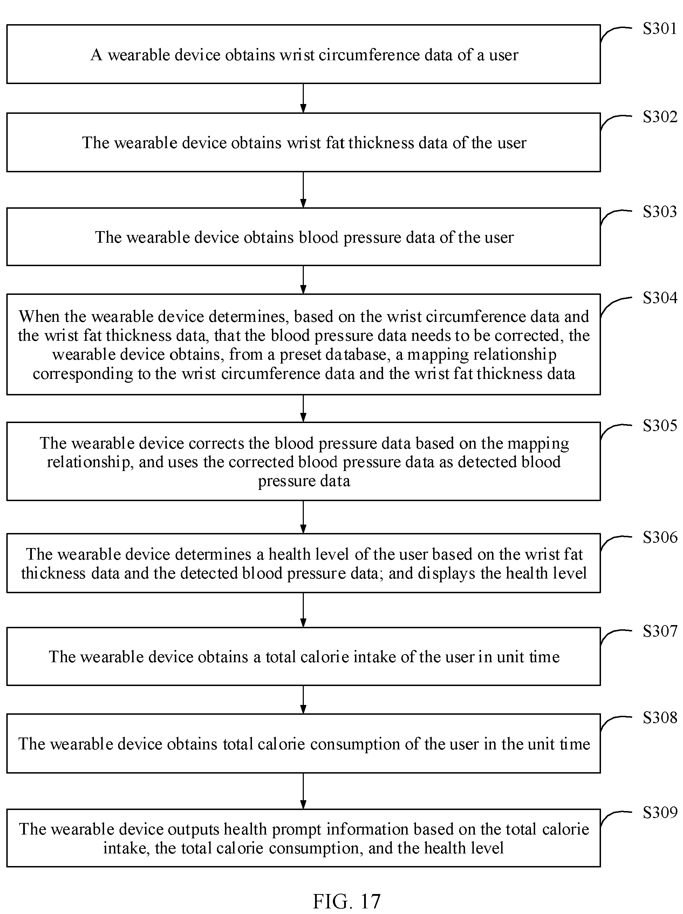

FIG. 17 is a schematic flowchart of another blood pressure detection method according to an embodiment of this application. Details are as follows:

S301: A wearable device obtains wrist circumference data of a user.

S302: The wearable device obtains wrist fat thickness data of the user.

S303: The wearable device obtains blood pressure data of the user.

S304: When the wearable device determines, based on the wrist circumference data and the wrist fat thickness data, that the blood pressure data needs to be corrected, the wearable device obtains, from a preset database, a mapping relationship corresponding to the wrist circumference data and the wrist fat thickness data.

S305: The wearable device corrects the blood pressure data based on the mapping relationship, and uses the corrected blood pressure data as detected blood pressure data.

S306: The wearable device determines a health level of the user based on the wrist fat thickness data and the detected blood pressure data; and displays the health level.

The health level may indicate a severe body problem, a large body problem, a slight body problem, and a good health condition.

Specifically, the health level corresponding to the blood pressure data and the wrist fat thickness data may be obtained from a third preset database.

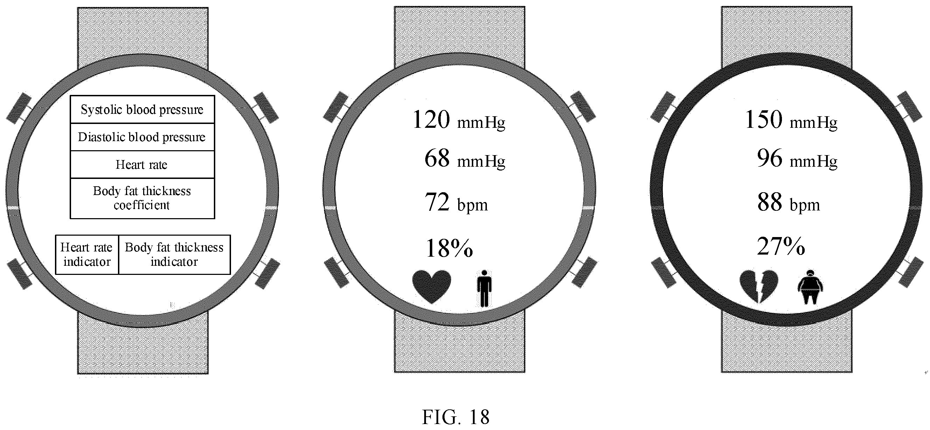

Optionally, coefficients such as a health level, target blood pressure, a heart rate, and a body fat thickness may be displayed in a manner of a chart, a table, an animation, and/or a text. For example, a schematic diagram of an interface for displaying a health level is shown in FIG. 18. A broken heart indicates that the user has arrhythmia, and a complete heart indicates that the user does not have arrhythmia. An icon of a thinner person indicates that the user has normal body fat, and an icon of a fatter person indicates that the user has relatively high body fat. Numbers from top to bottom indicate coefficients of systolic blood pressure, diastolic blood pressure, a heart rate, and a body fat thickness of the user. It should be noted that the foregoing body fat thickness coefficient may include a body fat percentage.

For example, LEDs may blink or glow to indicate whether the target blood pressure, the heart rate, and the body fat thickness coefficient of the user are normal. Alternatively, different health levels may be indicated by using colors (red, orange, yellow, green, and the like) of the LEDs. For example, a red LED indicates a serious problem of the body of the user, an orange LED indicates a relatively large problem of the body of the user, a yellow LED indicates a slight problem of the body of the user, and a green LED indicates a good health condition of the body of the user.

S307: The wearable device obtains a total calorie intake of the user in unit time.

Specifically, the total calorie intake that is of the user in the unit time and that is sent by a terminal may be obtained by using a wireless communications link. The total calorie intake of the user in the unit time is calculated based on a food image that is of the user in meals and that is photographed by the terminal and calories contained in each food. The terminal is connected to the electronic device, converts a dietary record of the user into the total calorie intake, and sends the total calorie intake to the electronic device.

S308: The wearable device obtains total calorie consumption of the user in the unit time.

For example, motion status information of the user in the unit time may be obtained by using a motion detection sensor, and the total calorie consumption of the user may be calculated based on the motion status information.

S309: The wearable device outputs health prompt information based on the total calorie intake, the total calorie consumption, and the health level.

A difference between a total calorie intake value and total calorie consumption is used as a net calorie consumption value. A level difference is obtained based on a historical health level and the health level. The health prompt information corresponding to the net calorie consumption value and the level difference is obtained. The health prompt information is displayed. The health prompt information includes a health improvement level and a health improvement suggestion.

It should be understood that sequence numbers of the steps do not mean execution sequences in the foregoing embodiments. The execution sequences of the processes should be determined based on functions and internal logic of the processes, and should not be construed as any limitation on the implementation processes of embodiments of this application.

The following describes embodiments of the present invention in detail with a specific example. The invention is defined by the appended claims.

It should be understood that the blood pressure watch in this example corresponds to the blood pressure detection apparatus in FIG. 14.

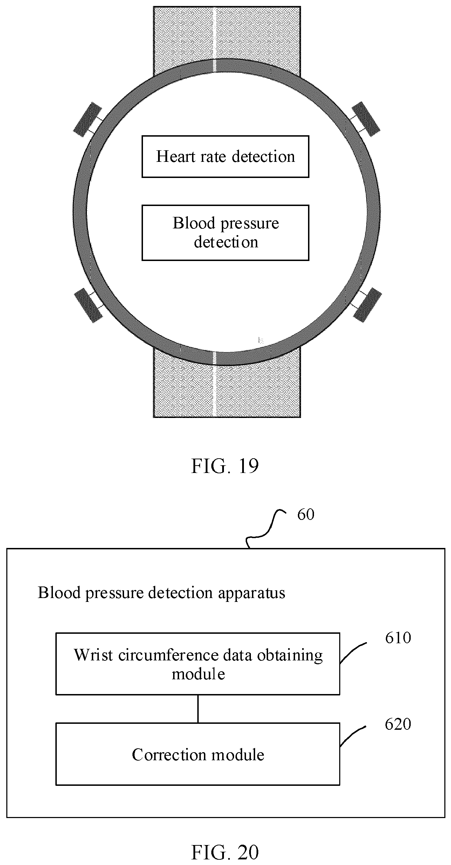

S190: A user presses a preset button of the blood pressure watch shown in FIG. 19, or a user triggers a "blood pressure detection" key in an interface of the blood pressure watch shown in FIG. 19. The blood pressure watch generates a detection instruction according to a button operation or a key operation.

S191: The blood pressure watch first detects, according to the detection instruction, an electrical parameter of an adjustable component around a wrist of the user, and obtains wrist circumference data corresponding to the electrical parameter. Specifically, the user adjusts a position of a buckle or a butterfly clasp of a strap based on a wrist circumference. The electrical parameter of the adjustable component is determined based on the position of the buckle or the butterfly clasp of the strap. For example, the blood pressure watch determines that the wrist circumference data is 181 mm.

S192: The blood pressure watch then inputs test currents on a plurality of frequencies to the user; detects a plurality of electric potential differences formed in the human body by the test currents on the plurality of frequencies; and obtains bioelectrical impedance on different frequencies based on the plurality of test currents and the plurality of electric potential differences, and determines wrist fat thickness data based on the bioelectrical impedance on different frequencies. For example, the blood pressure watch determines that the wrist fat thickness data is 4.5 mm.

S193: The blood pressure watch further obtains blood pressure data of the user. Specifically, the blood pressure watch obtains systolic blood pressure of the user: 140 mmHg, and obtains diastolic blood pressure of the user: 90 mmHg.

S194: The blood pressure watch determines, based on the wrist circumference data and the wrist fat thickness data, whether the blood pressure data needs to be corrected. Specifically, the blood pressure watch determines that the wrist fat thickness data is less than a second preset wrist fat thickness (for example, 5 mm) and greater than a first preset wrist fat thickness (for example, 2.5 mm), and that the wrist circumference data is greater than a second wrist circumference (for example, 180 mm). The blood pressure watch determines that the blood pressure data needs to be corrected.

S195: The blood pressure watch obtains, from a preset database, a fitting function corresponding to the wrist circumference data and the wrist fat thickness data.

S196: The blood pressure watch obtains a compensation value 4 mmHg by using a fitting function, corrects the blood pressure data based on the compensation value, uses the corrected blood pressure data (the systolic blood pressure of the user is 144 mmHg, and the diastolic blood pressure of the user is 94 mmHg) as detected blood pressure data, and displays the detected blood pressure data.

S197: The blood pressure watch determines a health level of the user based on the wrist fat thickness data and the detected blood pressure data, for example, 4; and displays the health level. For example, the health level is displayed on the interface. An orange LED is used to indicate a relatively large problem of the body of the user.

S198: The blood pressure watch obtains, by using a wireless communications link, a total calorie intake that is of the user within one day and that is sent by a terminal: 3200 Ka, and the blood pressure watch further obtains, by using a motion sensor, total calorie consumption of the user in unit time: 1589 Ka.

S199: The blood pressure watch uses a difference 1611 Ka between a total calorie intake value and total calorie consumption as a net calorie consumption value; and obtains a level difference: 1 based on a historical health level (level 3) and the health level, and obtains health prompt information corresponding to the net calorie consumption value 1611 Ka and the level difference: 1. For example, the health prompt information includes immediately measuring blood pressure by using a standard sphygmomanometer, taking a related medicament, reducing a calorie intake, and contacting a doctor. The blood pressure watch displays the health prompt information.

In correspondence with the blood pressure detection method in the foregoing embodiments, FIG. 20 is a block diagram of a structure of a blood pressure detection apparatus according to an embodiment of this application. For ease of description, only a part related to embodiments of this application is shown.

With reference to FIG. 20, the blood pressure detection apparatus 60 includes a wrist circumference data obtaining module 610 and a correction module 620.

The wrist circumference data obtaining module 610 is configured to obtain wrist circumference data, wrist fat thickness data, and blood pressure data.

The correction module 620 is configured to correct the blood pressure data based on the wrist circumference data and the wrist fat thickness data.

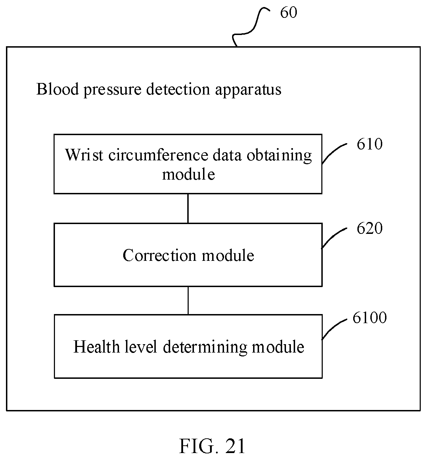

As shown in FIG. 21, the blood pressure detection apparatus 60 further includes a health level determining module 6100.

The health level determining module 6100 is configured to: determine a health level of a user based on the wrist fat thickness data and the detected blood pressure data; and display the health level.

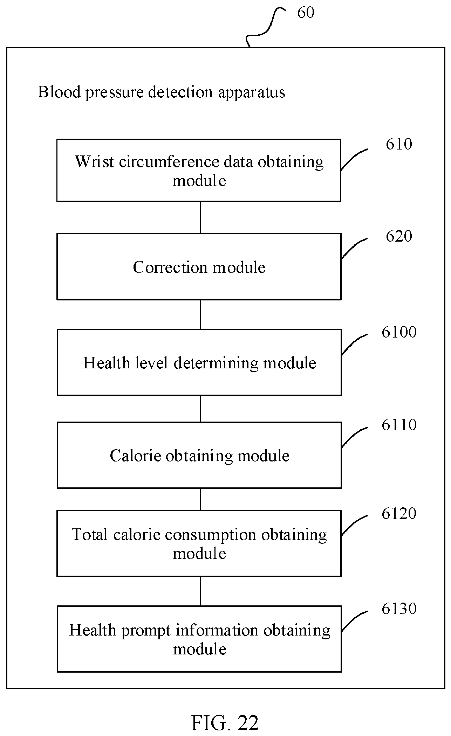

As shown in FIG. 22, the blood pressure detection apparatus 60 further includes a calorie obtaining module 6110, a total calorie consumption obtaining module 6120, and a health prompt information obtaining module 6130.

The calorie obtaining module 6110 is configured to obtain a total calorie intake of the user in unit time.

The total calorie consumption obtaining module 6120 is configured to obtain total calorie consumption of the user in the unit time.

The health prompt information obtaining module 6130 is configured to output health prompt information based on the total calorie intake, the total calorie consumption, and the health level.

The wrist circumference data obtaining module 610 includes an electrical parameter detection module 611a and a wrist circumference data determining module 612a.

The electrical parameter detection module 611a is configured to detect an electrical parameter of an adjustable component around a wrist of the user. The adjustable component includes at least one of an adjustable resistor, an adjustable capacitor, or an adjustable inductor.

The wrist circumference data determining module 612a is configured to obtain wrist circumference data corresponding to the electrical parameter.

In an implementation, the wrist circumference data obtaining module 610 is further specifically configured to perform impedance detection on the wrist of the user to obtain the wrist fat thickness data. The wrist circumference data obtaining module 610 further includes:

a test current input module 611b, configured to input test currents on a plurality of frequencies to the user; an electric potential difference detection module 612b, configured to detect a plurality of electric potential differences formed in a human body by the test currents on the plurality of frequencies; and a first wrist fat thickness data determining module 613b, configured to determine the wrist fat thickness data based on the plurality of test currents and the plurality of electric potential differences.

In another implementation, the wrist circumference data obtaining module 610 is further specifically configured to perform ultrasonic distance detection on the wrist of the user to obtain the wrist fat thickness data. The wrist circumference data obtaining module 610 further includes:

a first ultrasonic wave transmit module 611c, configured to: transmit a first ultrasonic wave to the wrist of the user, and record transmit time of the first ultrasonic wave;
a second ultrasonic wave capture module 612c, configured to: when the second ultrasonic wave is received, record receive time of the second ultrasonic wave, where the second ultrasonic wave is an ultrasonic wave reflected after the first ultrasonic wave reaches a bone of the wrist; and
a second wrist fat thickness data determining module 613c, configured to determine the wrist fat thickness data based on the transmit time of the first ultrasonic wave and the receive time of the second ultrasonic wave.

As shown in FIG. 23, the correction module 620 includes a mapping relationship obtaining module 621 and a display module 622.

The mapping relationship obtaining module 621 is configured to: determine, based on the wrist circumference data and the wrist fat thickness data, that the blood pressure data needs to be corrected, and obtain, from a preset database, a mapping relationship corresponding to the wrist circumference data and the wrist fat thickness data.

The display module 622 is configured to: correct the blood pressure data based on the mapping relationship, and display the corrected blood pressure data.

As shown in FIG. 24, the correction module 620 further includes a blood pressure determining module 623.

The blood pressure determining module 623 is configured to: if the blood pressure data does not need to be corrected, display the blood pressure data.

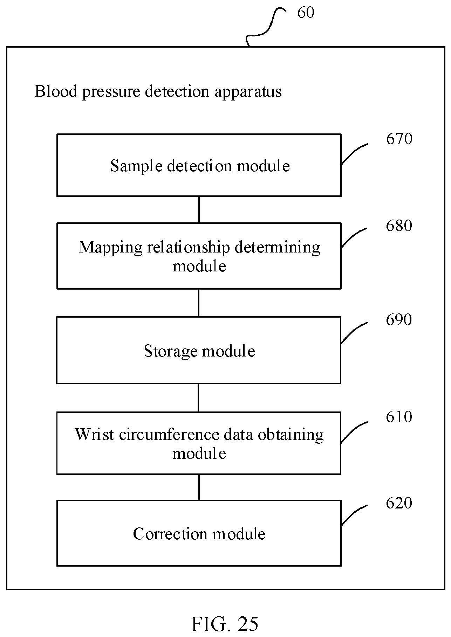

As shown in FIG. 25, the blood pressure detection apparatus 60 further includes a sample detection module 670, a mapping relationship determining module 680, and a storage module 690.

The sample detection module 670 is configured to obtain wrist circumference data, wrist fat thickness data, original blood pressure data, and standard blood pressure data of different figures of people.

The mapping relationship determining module 680 is configured to perform fitting on the wrist circumference data, the wrist fat thickness data, the original blood pressure data, and the standard blood pressure data of each figure of people to obtain a corresponding mapping relationship.

The storage module 690 is configured to store, in a database, the wrist circumference data, the wrist fat thickness data, and the corresponding mapping relationship of each figure of people.

It should be noted that content such as information exchange and an execution process between the apparatuses/units is based on the same idea as the method embodiments of this application. Therefore, for specific functions and beneficial effects, refer to the method embodiments. Details are not further described herein again.

It may be clearly understood by persons skilled in the art that, for the purpose of convenient and brief description, division of the foregoing function units and modules is used as an example for illustration. In actual application, the foregoing functions can be allocated to different function units and modules and implemented based on a requirement, that is, an inner structure of the apparatus is divided into different function units or modules to implement all or some of the functions described above. Function units and modules in embodiments may be integrated into one processing unit, or each of the units may exist alone physically, or two or more units are integrated into one unit. The integrated unit may be implemented through hardware, or may be implemented in a form of software function unit. The invention is defined by the appended claims.

For a detailed working process of the foregoing units and modules in the system, refer to a corresponding process in the foregoing method embodiments. Details are not described herein again. An embodiment of this application further provides a computer-readable storage medium. The computer-readable storage medium stores a computer program. The computer program may be executed by a processor to implement steps in the method embodiments.

An embodiment of this application provides a computer program product. When the computer program product is run on a mobile electronic device, the electronic device is enabled to perform the steps in the method embodiments during execution.

When the integrated unit is implemented in a form of a software function unit and sold or used as an independent product, the integrated unit may be stored in a computer-readable storage medium. Based on such understanding, all or some of the processes of the methods in embodiments in this application may be implemented by a computer program instructing related hardware. The computer program may be stored in a computer-readable storage medium. When the computer program is executed by a processor, the steps of the method embodiments may be implemented. The computer program includes computer program code. The computer program code may be in a source code form, an object code form, an executable file, some intermediate forms, or the like. The computer-readable medium may include at least any entity or apparatus that can carry the computer program code in the electronic device, a recording medium, a computer memory, a read-only memory (ROM, Read-Only Memory), a random access memory (RAM, Random Access Memory), an electrical carrier signal, a telecommunications signal, and a software distribution medium, for example, a USB flash drive, a removable hard disk, a magnetic disk, or an optical disc. In some jurisdictions, according to legislation and patent practice, the computer-readable medium cannot be an electrical carrier signal or a telecommunications signal.

In the foregoing embodiments, the description of each embodiment has respective focuses. For a part that is not described in detail in an embodiment, refer to related descriptions in other embodiments.

Persons of ordinary skill in the art may be aware that, in combination with the examples described in embodiments disclosed in this specification, units and algorithm steps may be implemented by electronic hardware or a combination of computer software and electronic hardware. Whether the functions are performed by hardware or software depends on particular applications and design constraint conditions of the technical solutions. Persons skilled in the art may use different methods to implement the described functions for each particular application, but it should not be considered that the implementation goes beyond the scope of this application.

In embodiments provided in this application, it should be understood that the disclosed apparatus/network device and method may be implemented in other manners. For example, the described apparatus/network device embodiment is merely an example. For example, the division into modules and units is merely logical function division and may be other division in actual implementation. For example, a plurality of units or components may be combined or integrated into another system, or some features may be ignored or not performed. In addition, the displayed or discussed mutual couplings or direct couplings or communication connections may be implemented through some interfaces. The indirect couplings or communication connections between the apparatuses or units may be implemented in electronic, mechanical, or other forms.

The units described as separate parts may or may not be physically separate, and parts displayed as units may or may not be physical units, may be located in one position, or may be distributed on a plurality of network units. Some or all of the units may be selected based on actual requirements to achieve the objectives of the solutions of embodiments. The foregoing embodiments are merely intended for describing the technical solutions of this application, but not for limiting this application. The intention is defined by the appended claims.

**Claims**

1. A blood pressure detection method, comprising:

   obtaining, by a wearable device, wrist circumference data, wrist fat thickness data, and blood pressure data; and
   correcting, by the wearable device, the blood pressure data based on the wrist circumference data and the wrist fat thickness data,
   wherein the obtaining wrist fat thickness data comprises:
   performing impedance detection, comprising:

   inputting test currents on a plurality of frequencies to the wrist of the user;
   detecting a plurality of electric potential differences formed at the wrist by the test currents on a plurality of frequencies; and
   determining the wrist fat thickness data based on the plurality of test currents and the plurality of electric potential differences.

2. The blood pressure detection method according to claim 1, wherein the obtaining wrist circumference data comprises:

   detecting an electrical parameter of an adjustable component (031) around a wrist of a user, wherein the adjustable component (031) comprises at least one of an adjustable resistor, an adjustable capacitor, or an adjustable inductor; and
   obtaining the wrist circumference data corresponding to the electrical parameter.

3. The blood pressure detection method according to claim 1, wherein after the correcting, by the wearable device, the blood pressure data based on the wrist circumference data and the wrist fat thickness data, the method further comprises:

   determining, by the wearable device, a health level of the user based on the wrist fat thickness data and the detected blood pressure data; and
   displaying, by the wearable device, the health level.

4. The blood pressure detection method according to claim 3, wherein after determining the health level of the user, the method further comprises:

   obtaining, by the wearable device, a total calorie intake of the user within unit time;
   obtaining, by the wearable device, total calorie consumption of the user within the unit time; and
   outputting, by the wearable device, health prompt information based on the total calorie intake, the total calorie consumption, and the health level.

5. The blood pressure detection method according to claim 1, wherein the correcting, by the wearable device, the blood pressure data based on the wrist circumference data and the wrist fat thickness data comprises:

   when determining, based on the wrist circumference data and the wrist fat thickness data, that the blood pressure data needs to be corrected, obtaining, by the wearable device from a preset database, a mapping relationship corresponding to the wrist circumference data and the wrist fat thickness data, wherein the mapping relationship is the mapping relationship corresponding to the wrist circumference data and the wrist fat thickness data; and
   correcting, by the wearable device, the blood pressure data based on the mapping relationship, and displaying corrected blood pressure data.

6. A blood pressure detection apparatus, comprising:

   a main body part and a strap connected to the main body part, wherein the strap is configured to wear the main body part on a wrist of the user; and
   a wrist circumference detection component (03), a wrist fat thickness detection component (01), a blood pressure detection component (02), and a processor (04), wherein the processor (04) is disposed in the main body part and is separately connected to the wrist circumference detection component (03), the wrist fat thickness detection component (01), and the blood pressure detection component (02);
   the wrist circumference detection component (03) is configured to obtain wrist circumference data of a user;
   the wrist fat thickness detection component (01) is configured to obtain wrist fat thickness data of the user, wherein the wrist fat thickness detection component (01) comprises:

   an excitation electrode (011), a detection electrode (012), and an impedance detection circuit (013), wherein

the excitation electrode (011) is disposed on a side surface of the main body part and is configured to forward an excitation voltage; the detection electrode (012) is disposed at a bottom of the main body part and is configured to receive a detection voltage, wherein the detection voltage is generated based on the excitation voltage and a voltage drop of wrist fat of the user; and the impedance detection circuit (013) is disposed in the main body part and is configured to: generate the excitation voltage, and obtain the wrist fat thickness data through calculation based on the excitation voltage and the detection voltage; the blood pressure detection component (02) is configured to obtain blood pressure data of the user; and the processor (04) is configured to correct the blood pressure data based on the wrist circumference data and the wrist fat thickness data.

7. The blood pressure detection apparatus according to claim 6, wherein the wrist circumference detection component (03) comprises:

an adjustable component (031) and an electrical parameter detection circuit (032), wherein the adjustable component (031) is connected to the electrical parameter detection circuit (032), and the electrical parameter detection circuit (032) is disposed in the main body part and is configured to: detect an electrical parameter of the adjustable component (031), and obtain, based on a preset correspondence between the electrical parameter and the wrist circumference data, wrist circumference data corresponding to the detected electrical parameter.

8. The blood pressure detection apparatus according to claim 7, wherein the strap comprises a first strap (201-1) and a second strap (201-2), and the first strap (201-1) and the second strap (201-2) are separately connected to the main body part;

the adjustable component (031) is disposed on a first surface of the first strap (201-1), and the first surface is a surface close to the wrist when the blood pressure detection apparatus is worn on the wrist of the user; the first strap (201-1) is further provided with a plurality of holes (203) parallel to the adjustable component (031), and the adjustable component (031) is connected to the holes (203) through a first conducting wire (202); and the second strap (201-2) is provided with a buckle tongue (209) matching the holes (203), a second conducting wire (205) is disposed on both surfaces of the buckle tongue (209) and the holes (203), and when the buckle tongue (209) is connected to the holes (203) through the second conducting wire (205), an electrical parameter of the adjustable component (031) may be adjusted based on a position of the buckle tongue (209).

9. The blood pressure detection apparatus according to claim 7, wherein the strap comprises a first strap (201-1) and a second strap (201-2), and the first strap (201-1) and the second strap (201-2) are separately connected to the main body part;

the adjustable component (031) is disposed on a first surface of the first strap (201-1), the first surface is a surface close to the wrist when the blood pressure detection apparatus is worn on the wrist of the user, an insulator (212) is disposed on a surface of the adjustable component (031), and a plurality of third conducting wires (216) are embedded in the insulator (212) in a spaced manner and connected to the adjustable component (031); and the second strap (201-2) is provided with a movable butterfly clasp (213), the butterfly clasp (213) is connected to the adjustable component (031) through the third conducting wires (216), and when the butterfly clasp (213) moves on the strap, an electrical parameter of the adjustable component (031) may be adjusted based on a position of the butterfly clasp (213).

10. The blood pressure detection apparatus according to any one of claims 7 to 9, wherein the adjustable component (031) comprises at least one of an adjustable resistor, an adjustable capacitor, and an adjustable inductor.

11. The blood pressure detection apparatus according to claim 6, wherein there are a plurality of excitation electrodes (011), and the excitation electrodes (011) are sequentially arranged on the side surface of the main body part; and

there are a plurality of detection electrodes (012), and the detection electrodes (012) are disposed at the bottom of the main body part and are sequentially arranged along the periphery of the bottom of the main body part, wherein each excitation electrode (011) is connected to one detection electrode (012).

12. The blood pressure detection apparatus according to claim 6, wherein the blood pressure detection component (02) comprises:

an air pump, an airbag (220), and a pressure sensor, wherein

the air pump and the pressure sensor are disposed in the main body part, the airbag (220) is disposed on a second surface of the strap, and the second surface is a contact surface of the strap with the wrist when the blood pressure detection apparatus is worn on the wrist of the user;

the air pump is connected to the airbag (220), and the airbag (220) is connected to the pressure sensor;

the air pump is configured to inflate the airbag (220); and

the pressure sensor is configured to: in a process of inflating the airbag (220), detect air pressure in the airbag (220) in real time, and obtain the blood pressure data through calculation based on the air pressure.

## Patentansprüche

1. Blutdruckdetektionsverfahren, umfassend:

   Erhalten von Handgelenkumfangsdaten, Handgelenkfettdickendaten und Blutdruckdaten durch eine tragbare Vorrichtung; und
   Korrigieren der Blutdruckdaten durch die tragbare Vorrichtung basierend auf den Handgelenkumfangsdaten und den Handgelenkfettdickendaten,
   wobei das Erhalten von Handgelenkfettdickendaten Folgendes umfasst:
   Durchführen von Impedanzdetektion, umfassend:

   Eingeben von Testströmen auf einer Mehrzahl von Frequenzen in das Handgelenk des Benutzers;
   Detektieren einer Mehrzahl von elektrischen Potenzialdifferenzen, die an dem Handgelenk durch die Testströme auf einer Mehrzahl von Frequenzen gebildet wird; und
   Bestimmen der Handgelenkfettdickendaten basierend auf der Mehrzahl von Testströmen und der Mehrzahl von elektrischen Potenzialdifferenzen.

2. Blutdruckdetektionsverfahren nach Anspruch 1, wobei das Erhalten von Handgelenkumfangsdaten Folgendes umfasst:

   Detektieren eines elektrischen Parameters einer einstellbaren Komponente (031) um ein Handgelenk eines Benutzers, wobei die einstellbare Komponente (031) mindestens eines von einem einstellbaren Widerstand, einem einstellbaren Kondensator und einem einstellbaren Induktor umfasst; und
Erhalten der Handgelenkumfangsdaten, die dem elektrischen Parameter entsprechen.

3. Blutdruckdetektionsverfahren nach Anspruch 1, wobei das Verfahren nach dem Korrigieren der Blutdruckdaten durch die tragbare Vorrichtung basierend auf den Handgelenkumfangsdaten und den Handgelenkfettdickendaten ferner Folgendes umfasst:

   Bestimmen eines Gesundheitszustands des Benutzers durch die tragbare Vorrichtung basierend auf den Handgelenkfettdickendaten und den detektierten Blutdruckdaten; und
   Anzeigen des Gesundheitszustands durch die tragbare Vorrichtung.

4. Blutdruckdetektionsverfahren nach Anspruch 3, wobei das Verfahren nach dem Bestimmen des Gesundheitszustands des Benutzers ferner Folgendes umfasst:

   Erhalten der Gesamtkalorienaufnahme des Benutzers innerhalb einer Zeiteinheit durch die tragbare Vorrichtung;
   Erhalten des Gesamtkalorienverbrauchs des Benutzers innerhalb der Zeiteinheit durch die tragbare Vorrichtung; und
   Ausgeben von Gesundheitsanzeigeinformationen durch die tragbare Vorrichtung basierend auf der Gesamtkalorienaufnahme, dem Gesamtkalorienverbrauch und dem Gesundheitszustand.

5. Blutdruckdetektionsverfahren nach Anspruch 1, wobei das Korrigieren der Blutdruckdaten durch die tragbare Vorrichtung basierend auf den Handgelenkumfangsdaten und den Handgelenkfettdickendaten Folgendes umfasst:

   wenn basierend auf den Handgelenkumfangsdaten und den Handgelenkfettdickendaten bestimmt wird, dass der Blutdruck korrigiert werden muss, Erhalten einer Zuordnungsbeziehung, die den Handgelenkumfangsdaten und den Handgelenkfettdickendaten entspricht, durch die tragbare Vorrichtung aus einer voreingestellten Datenbank, wobei die Zuordnungsbeziehung die Zuordnungsbeziehung ist, die den Handgelenkumfangsdaten und den Handgelenkfettdickendaten entspricht; und
   Korrigieren der Blutdruckdaten durch die tragbare Vorrichtung basierend auf der Zuordnungsbeziehung und Anzeigen der korrigierten Blutdruckdaten.

6. Blutdruckdetektionseinrichtung, umfassend:

einen Hauptkörperteil und ein mit dem Hauptkörperteil verbundenes Band, wobei das Band dazu ausgelegt ist, den Hauptkörperteil auf einem Handgelenk des Benutzers zu tragen; und eine Handgelenkumfangsdetektionskomponente (03), eine Handgelenkfettdickendetektionskomponente (01), eine Blutdruckdetektionskomponente (02) und einen Prozessor (04), wobei der Prozessor (04) in dem Hauptkörperteil angeordnet und separat mit der Handgelenkumfangsdetektionskomponente (03), der Handgelenkfettdickendetektionskomponente (01) und der Blutdruckdetektionskomponente (02) verbunden ist;
wobei die Handgelenkumfangsdetektionskomponente (03) dazu ausgelegt ist, Handgelenkumfangsdaten eines Benutzers zu erhalten;
die Handgelenkfettdickendetektionskomponente (01) dazu ausgelegt ist, Handgelenkfettdickendaten des Benutzers zu erhalten,
wobei die Handgelenkfettdickendetektionskomponente (01) Folgendes umfasst:

eine Anregungselektrode (011), eine Detektionselektrode (012) und eine Impedanzdetektionsschaltung (013), wobei
die Anregungselektrode (011) auf einer Seitenfläche des Hauptkörperteils angeordnet und zum Weiterleiten einer Anregungsspannung ausgelegt ist;
die Detektionselektrode (012) auf einer Unterseite des Hauptkörperteils angeordnet und zum Empfangen einer Detektionsspannung ausgelegt ist, wobei die Detektionsspannung basierend auf der Anregungsspannung und einem Spannungsabfall des Handgelenkfetts des Benutzers erzeugt wird; und
die Impedanzdetektionsschaltung (013) in dem Hauptkörperteil angeordnet und zu Folgendem ausgelegt ist: Erzeugen der Anregungsspannung und Erhalten der Handgelenkfettdickendaten durch Berechnung basierend auf der Anregungsspannung und der Detektionsspannung;
die Blutdruckdetektionskomponente (02) dazu ausgelegt ist, Blutdruckdaten des Benutzers zu erhalten; und
der Prozessor (04) dazu ausgelegt ist, die Blutdruckdaten basierend auf den Handgelenkumfangsdaten und den Handgelenkfettdickendaten zu korrigieren.

7. Blutdruckdetektionseinrichtung nach Anspruch 6, wobei die Handgelenkumfangskomponente (03) Folgendes umfasst:

eine einstellbare Komponente (031) und eine Schaltung zur Detektion elektrischer Parameter (032), wobei die einstellbare Komponente (031) mit der Schaltung zur Detektion elektrischer Parameter (032) verbunden ist, und
die Schaltung zur Detektion elektrischer Parameter (032) in dem Hauptkörperteil angeordnet und zu Folgendem ausgelegt ist: Detektieren eines elektrischen Parameters der einstellbaren Komponente (031) und Erhalten von Handgelenkumfangsdaten, die dem elektrischen Parameter entsprechen, basierend auf einer voreingestellten Entsprechung zwischen dem elektrischen Parameter und den Handgelenkumfangsdaten.

8. Blutdruckdetektionseinrichtung nach Anspruch 7, wobei das Band ein erstes Band (201-1) und ein zweites Band (201-2) umfasst und das erste Band (201-1) und das zweite Band (201-2) separat mit dem Hauptkörperteil verbunden sind;

die einstellbare Komponente (031) auf einer ersten Oberfläche des ersten Bandes (201-1) angeordnet ist und die erste Oberfläche eine Oberfläche in der Nähe des Handgelenks ist, wenn die Blutdruckdetektionseinrichtung am Handgelenk des Benutzers getragen wird;
das erste Band (201-1) ferner mit einer Mehrzahl von Löchern (203) parallel zur einstellbaren Komponente (031) versehen ist und die einstellbare Komponente (031) über einen ersten Leitungsdraht (202) mit den Löchern (203) verbunden ist; und
das zweite Band (201-2) mit einer Schlosszunge (209) versehen ist, die mit den Löchern (203) zusammenpasst, wobei ein zweiter Leitungsdraht (205) auf beiden Oberflächen der Schlosszunge (209) und der Löcher (203) angeordnet ist und, wenn die Schlosszunge (209) über den zweiten Leitungsdraht (205) mit den Löchern (203) verbunden ist, ein elektrischer Parameter der einstellbaren Komponente (031) basierend auf einer Position der Schlosszunge (209) eingestellt werden kann.

9. Blutdruckdetektionseinrichtung nach Anspruch 7, wobei das Band ein erstes Band (201-1) und ein zweites Band (201-2) umfasst und das erste Band (201-1) und das zweite Band (201-2) separat mit dem Hauptkörperteil verbunden sind;

die einstellbare Komponente (031) auf einer ersten Oberfläche des ersten Bandes (201-1) angeordnet ist, die erste Oberfläche eine Oberfläche in der Nähe des Handgelenks ist, wenn die Blutdruckdetektionseinrichtung auf dem Handgelenk des Benutzers getragen wird, ein Isolator (212) auf einer Oberfläche der einstellbaren Komponente (031) angeordnet ist und eine Mehrzahl von dritten Leitungsdrähten (216) in den Isolator (212) in einer beabstandeten Weise eingebettet und mit der einstellbaren Komponente (031) verbunden sind; und

das zweite Band (201-2) mit einem beweglichen Schmetterlingsverschluss (213) versehen ist, der Schmetterlingsverschluss (213) über die dritten Leitungsdrähte (216) mit der einstellbaren Komponente (031) verbunden ist und, wenn sich der Schmetterlingsverschluss (213) auf dem Band bewegt, ein elektrischer Parameter der einstellbaren Komponente (031) basierend auf einer Position des Schmetterlingsverschlusses (213) eingestellt werden kann.

**10.** Blutdruckdetektionseinrichtung nach einem der Ansprüche 7 bis 9, wobei die einstellbare Komponente (031) mindestens eines von einem einstellbaren Widerstand, einem einstellbaren Kondensator und einem einstellbaren Induktor umfasst.

**11.** Blutdruckdetektionseinrichtung nach Anspruch 6, wobei eine Mehrzahl von Anregungselektroden (011) vorhanden ist und die Anregungselektroden (011) der Reihe nach auf der Seitenfläche des Hauptkörperteils angeordnet sind; und

eine Mehrzahl von Detektionselektroden (012) vorhanden ist und die Detektionselektroden (012) auf der Unterseite des Hauptkörperteils angeordnet sind und der Reihe nach entlang des Umfangs der Unterseite des Hauptkörperteils angeordnet sind, wobei

jede Anregungselektrode (011) mit einer Detektionselektrode (012) verbunden ist.

**12.** Blutdruckdetektionseinrichtung nach Anspruch 6, wobei die Blutdruckdetektionskomponente (02) Folgendes umfasst:

eine Luftpumpe, einen Airbag (220) und einen Drucksensor, wobei die Luftpumpe und der Drucksensor in dem Hauptkörperteil angeordnet sind, der Airbag (220) auf einer zweiten Oberfläche des Bandes angeordnet ist und die zweite Oberfläche eine Kontaktfläche des Bandes mit dem Handgelenk ist, wenn die Blutdruckdetektionseinrichtung auf dem Handgelenk des Benutzers getragen wird; die Luftpumpe mit dem Airbag (220) verbunden ist und der Airbag (220) mit dem Drucksensor verbunden ist; die Luftpumpe dazu ausgelegt ist, den Airbag (220) aufzublasen; und der Drucksensor zu Folgendem ausgelegt ist: in einem Prozess des Aufblasens des Airbags (220) Detektieren des Luftdrucks im Airbag (220) in Echtzeit und Erhalten der Blutdruckdaten durch Berechnung basierend auf dem Luftdruck.

**Revendications**

**1.** Procédé de détection de tension artérielle, comprenant :

l'obtention, par un dispositif portable, de données de circonférence de poignet, de données d'épaisseur de masse grasse de poignet, et de données de pression artérielle ; et
la correction, par le dispositif portable, des données de pression artérielle sur la base des données de circonférence de poignet et des données d'épaisseur de masse grasse de poignet, dans lequel l'obtention de données d'épaisseur de masse grasse de poignet comprend :
la réalisation d'une détection d'impédance, comprenant :
l'entrée de courants de test, sur une pluralité de fréquences, dans le poignet de l'utilisateur :

la détection d'une pluralité de différences de potentiel électrique formées au niveau du poignet par les courants de test sur une pluralité de fréquences ; et
la détermination des données d'épaisseur de masse grasse de poignet sur la base de la pluralité de courants de test et de la pluralité de différences de potentiel électrique.

**2.** Procédé de détection de tension artérielle selon la revendication l, dans lequel l'obtention de données de circonférence de poignet comprend :

la détection d'un paramètre électrique d'un composant réglable (031) autour d'un poignet d'un utilisateur, dans lequel le composant réglable (031) comprend au moins un élément parmi une résistance réglable, un condensateur réglable, ou un inducteur réglable ; et
l'obtention des données de circonférence de poignet correspondant au paramètre électrique.

**3.** Procédé de détection de tension artérielle selon la revendication l, dans lequel, après la correction, par le dispositif portable, des données de pression artérielle sur la base des données de circonférence de poignet et des données d'épaisseur de masse grasse de poignet, le procédé comprend en outre :

la détermination, par le dispositif portable, d'un niveau de santé de l'utilisateur sur la base des données d'épaisseur de masse grasse de poignet et des données de pression artérielle détectées ; et

l'affichage, par le dispositif portable, du niveau de santé.

**4.** Procédé de détection de tension artérielle selon la revendication 3, dans lequel, après la détermination du niveau de santé de l'utilisateur, le procédé comprend en outre :

l'obtention, par le dispositif portable, d'un apport calorique total de l'utilisateur dans les limites d'un temps unitaire ;

l'obtention, par le dispositif portable, d'une consommation calorique totale de l'utilisateur dans les limites du temps unitaire ; et

la sortie, par le dispositif portable, d'informations d'invite de santé sur la base de l'apport calorique total, de la consommation calorique totale, et du niveau de santé.

**5.** Procédé de détection de tension artérielle selon la revendication I, dans lequel la correction, par le dispositif portable, des données de pression artérielle sur la base des données de circonférence de poignet et des données d'épaisseur de masse grasse de poignet comprend :

lors de la détermination, sur la base des données de circonférence de poignet et des données d'épaisseur de masse grasse de poignet, que les données de pression artérielle doivent être corrigées, l'obtention, par le dispositif portable, auprès d'une base de données prédéfinie, d'une relation de mappage correspondant aux données de circonférence de poignet et aux données d'épaisseur de masse grasse de poignet, dans lequel la relation de mappage est la relation de mappage correspondant aux données de circonférence de poignet et aux données d'épaisseur de masse grasse de poignet ; et

la correction, par le dispositif portable, des données de pression artérielle sur la base de la relation de mappage, et l'affichage de données de pression artérielle corrigées.

**6.** Appareil de détection de tension artérielle, comprenant :

un partie corps principale et un bracelet raccordé à la partie corps principale, dans lequel le bracelet est configuré pour porter la partie corps principale sur un poignet de l'utilisateur ; et un composant de détection de circonférence de poignet (03), un composant de détection d'épaisseur de masse grasse de poignet (01), un composant de détection de pression artérielle (02), et un processeur (04), dans lequel le processeur (04) est disposé dans la partie corps principale et est séparément raccordé au composant de détection de circonférence de poignet (03), au composant de détection d'épaisseur de masse grasse de poignet (01), et au composant de détection de pression artérielle (02) ;

le composant de détection de circonférence de poignet (03) est configuré pour obtenir des données de circonférence de poignet d'un utilisateur ;

le composant de détection d'épaisseur de masse grasse de poignet (01) est configuré pour obtenir des données d'épaisseur de masse grasse de poignet de l'utilisateur, dans lequel le composant de détection d'épaisseur de masse grasse de poignet (01) comprend :

une électrode d'excitation (011), une électrode de détection (012), et un circuit de détection d'impédance (013), dans lequel l'électrode d'excitation (011) est disposée sur une surface latérale de la partie corps principale et est configurée pour envoyer une tension électrique d'excitation ;

l'électrode de détection (012) est disposée à un fond de la partie corps principale et est configurée pour recevoir une tension électrique de détection, dans lequel la tension électrique de détection est générée sur la base de la tension électrique d'excitation et d'une chute de tension électrique de masse grasse de poignet de l'utilisateur ; et

le circuit de détection d'impédance (013) est disposé dans la partie corps principale et est configuré pour : générer la tension électrique d'excitation, et obtenir les données d'épaisseur de masse grasse de poignet par le biais de calcul sur la base de la tension électrique d'excitation et de la tension électrique de détection ;

le composant de détection de pression artérielle (02) est configuré pour obtenir des données de pression artérielle de l'utilisateur ; et

le processeur (04) est configuré pour corriger les données de pression artérielle sur la base des données de circonférence de poignet et des données d'épaisseur de masse grasse de poignet.

**7.** Appareil de détection de tension artérielle selon la revendication 6, dans lequel le composant de détection de circonférence de poignet (03) comprend :

un composant réglable (031) et un circuit de détection de paramètre électrique (032), dans lequel le composant réglable (031) est raccordé au circuit de détection de paramètre électrique (032), et

le circuit de détection de paramètre électrique (032) est disposé dans la partie corps principale et est configuré pour : détecter un paramètre électrique du composant réglable (031), et obtenir, sur la base d'une correspondance prédéfinie entre le paramètre électrique et les données de circonférence de poignet, des données de circonférence de poignet correspondant au paramètre électrique détecté.

8. Appareil de détection de tension artérielle selon la revendication 7, dans lequel le bracelet comprend un premier bracelet (201-1) et un second bracelet (201-2), et le premier bracelet (201-1) et le second bracelet (201-2) sont séparément raccordés à la partie corps principale ;

le composant réglable (031) est disposé sur une première surface du premier bracelet (201-1), et la première surface est une surface proche du poignet lorsque l'appareil de détection de tension artérielle est porté sur le poignet de l'utilisateur ;

le premier bracelet (201-1) est en outre pourvu d'une pluralité de trous (203) parallèles au composant réglable (031), et le composant réglable (031) est raccordé aux trous (203) par le biais d'un premier fil conducteur (202) ; et

le second bracelet (201-2) est pourvu d'un ardillon de boucle (209) coïncidant avec les trous (203), un deuxième fil conducteur (205) est disposé sur les deux surfaces de l'ardillon de boucle (209) et les trous (203), et lorsque l'ardillon de boucle (209) est raccordé aux trous (203) par le biais du deuxième fil conducteur (205), un paramètre électrique du composant réglable (031) peut être réglé sur la base d'une position de l'ardillon de boucle (209).

9. Appareil de détection de tension artérielle selon la revendication 7, dans lequel le bracelet comprend un premier bracelet (201-1) et un second bracelet (201-2), et le premier bracelet (201-1) et le second bracelet (201-2) sont séparément raccordés à la partie corps principale ;

le composant réglable (031) est disposé sur une première surface du premier bracelet (201-1), la première surface est une surface proche du poignet lorsque l'appareil de détection de tension artérielle est porté sur le poignet de l'utilisateur, un isolant (212) est disposé sur une surface du composant réglable (031), et une pluralité de troisièmes fils conducteurs (216) sont encastrés dans l'isolant (212) de manière espacée et raccordés au composant réglable (031) ; et

le second bracelet (201-2) est pourvu d'un fermoir papillon mobile (213), le fermoir papillon (213) est raccordé au composant réglable (031) par le biais des troisièmes fils conducteurs (216), et, lorsque le fermoir papillon (213) se meut sur le bracelet, un paramètre électrique du composant réglable (031) peut être réglé sur la base d'une position du fermoir papillon (213).

10. Appareil de détection de tension artérielle selon l'une quelconque des revendications 7 à 9, dans lequel le composant réglable (031) comprend au moins un élément parmi une résistance réglable, un condensateur réglable, et un inducteur réglable.

11. Appareil de détection de tension artérielle selon la revendication 6, dans lequel il y a une pluralité d'électrodes d'excitation (011), et les électrodes d'excitation (011) sont agencées séquentiellement sur la surface latérale de la partie corps principale ; et
il y a une pluralité d'électrodes de détection (012), et les électrodes de détection (012) sont disposées au fond de la partie corps principale et sont séquentiellement agencées le long de la périphérie du fond de la partie corps principale, dans lequel chaque électrode d'excitation (011) est raccordée à une électrode de détection (012).

12. Appareil de détection de tension artérielle selon la revendication 6, dans lequel le composant de détection de pression artérielle (02) comprend :

une pompe à air, un coussinet à air (220), et un capteur de pression, dans lequel
la pompe à air et le capteur de pression sont disposés dans la partie corps principale, le coussinet à air (220) est disposé sur une seconde surface du bracelet, et la seconde surface est une surface de contact du bracelet avec le poignet lorsque l'appareil de détection de tension artérielle est porté sur le poignet de l'utilisateur ;
la pompe à air est raccordée au coussinet à air (220), et le coussinet à air (220) est raccordé au capteur de pression ;
la pompe à air est configurée pour gonfler le coussinet à air (220) ; et
le capteur de pression est configuré pour : dans un processus de gonflage du coussinet à air (220), détecter une pression de l'air dans le coussinet à air (220) en temps réel, et obtenir les données de pression artérielle par le biais de calcul sur la base de la pression d'air.

01

┌─────────────────┐
│ Wrist fat       │
│ thickness       │
│ detection       │
│ component       │
└─────────────────┘

02

┌─────────────────┐
│ Blood pressure  │
│ detection       │
│ component       │
└─────────────────┘

03

┌─────────────────┐
│ Wrist           │
│ circumference   │
│ detection       │
│ component       │
└─────────────────┘

04

┌─────────────────┐
│                 │
│                 │
│   Processor     │
│                 │
│                 │
└─────────────────┘

FIG. 1

031

┌─────────────────┐
│ Adjustable      │
│ component       │
└─────────────────┘

032

┌─────────────────┐
│ Electrical      │
│ parameter       │
│ detection circuit│
└─────────────────┘

FIG. 2

FIG. 3

FIG. 4

210

209

208

201-2

FIG. 5

205

206

207

FIG. 6

FIG. 7

FIG. 8

211-1

212

213

216

FIG. 9

```
011                                        013
┌──────────────┐              ┌──────────────┐
│  Excitation  │              │              │
│  electrode   │──────────────│              │
│              │              │              │
└──────────────┘              │              │
                              │  Impedance   │
                              │  detection   │
                              │   circuit    │
                              │              │
           012                │              │
┌──────────────┐              │              │
│  Detection   │──────────────│              │
│  electrode   │              │              │
│              │              │              │
└──────────────┘              └──────────────┘
```

FIG. 10

FIG. 11

FIG. 12

01

Wrist fat
thickness
detection
component

04

Processor

02

Blood pressure
detection
component

03

Wrist
circumference
detection
component

05

Indication
component

FIG. 13

A wearable device obtains wrist circumference data, wrist fat thickness data, and blood pressure data — S101

The wearable device corrects the blood pressure data based on the wrist circumference data and the wrist fat thickness data — S102

FIG. 14

FIG. 15

Obtain wrist circumference data, wrist fat thickness data, original blood pressure data, and standard blood pressure data of different figures of people — S201

Perform fitting on the wrist circumference data, the wrist fat thickness data, the original blood pressure data, and the standard blood pressure data of each figure of people to obtain a corresponding fitting function — S202

Store, in a database, the wrist circumference data, the wrist fat thickness data, and the corresponding fitting function of each figure of people — S203

A wearable device obtains wrist circumference data of a user — S204

The wearable device obtains wrist fat thickness data of the user — S205

The wearable device obtains blood pressure data of the user — S206

The wearable device determines, based on the wrist circumference data and the wrist fat thickness data, whether the blood pressure data needs to be corrected — S207

No

Yes

The wearable device uses the blood pressure data as detected blood pressure data — S208a

The wearable device obtains, from a preset database, a mapping relationship corresponding to the wrist circumference data and the wrist fat thickness data — S208b

The wearable device corrects the blood pressure data based on the mapping relationship, and uses the corrected blood pressure data as detected blood pressure data — S209b

FIG. 16

A wearable device obtains wrist circumference data of a user — S301

The wearable device obtains wrist fat thickness data of the user — S302

The wearable device obtains blood pressure data of the user — S303

When the wearable device determines, based on the wrist circumference data and the wrist fat thickness data, that the blood pressure data needs to be corrected, the wearable device obtains, from a preset database, a mapping relationship corresponding to the wrist circumference data and the wrist fat thickness data — S304

The wearable device corrects the blood pressure data based on the mapping relationship, and uses the corrected blood pressure data as detected blood pressure data — S305

The wearable device determines a health level of the user based on the wrist fat thickness data and the detected blood pressure data; and displays the health level — S306

The wearable device obtains a total calorie intake of the user in unit time — S307

The wearable device obtains total calorie consumption of the user in the unit time — S308

The wearable device outputs health prompt information based on the total calorie intake, the total calorie consumption, and the health level — S309

FIG. 17

FIG. 18

FIG. 19

FIG. 20

FIG. 21

60

Blood pressure detection apparatus

Wrist circumference data obtaining module — 610

Correction module — 620

Health level determining module — 6100

Calorie obtaining module — 6110

Total calorie consumption obtaining module — 6120

Health prompt information obtaining module — 6130

FIG. 22

620

Correction module

Mapping relationship obtaining module — 621

Display module — 622

FIG. 23

620

Correction module

Mapping relationship obtaining module — 621

Display module — 622

Blood pressure determining module — 623

FIG. 24

60

Blood pressure detection apparatus

Sample detection module — 670

Mapping relationship determining module — 680

Storage module — 690

Wrist circumference data obtaining module — 610

Correction module — 620

FIG. 25

**EP 4 129 164 B1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202010301533
- US 2019365259 A1
- US 2014163399 A1
- US 2019290143 A1
- US 2010036265 A1
- JP 2002159457 A
- CN 207152582U
- US 2016038055 A1
- US 2003149369 A1